# EUROPÄISCHE PATENTSCHRIFT

(11) **EP 3 153 128 B1**
(45) Veröffentlichungstag und Bekanntmachung des Hinweises auf die Patenterteilung: **14.11.2018**
(21) Anmeldenummer: 16002049.1
(22) Anmeldetag: 22.09.2016
(51) Int. Cl.: A61B 50/30

(54) **VERPACKUNGSHÜLSE FÜR MEDIZINISCHE ZWECKE**
PACKAGING SLEEVE FOR MEDICINAL PURPOSES
ÉTUI D'EMBALLAGE À DES FINS MÉDICALES

(30) Priorität: 06.10.2015 DE 102015012898
(43) Veröffentlichungstag der Anmeldung: 12.04.2017
(73) Patentinhaber: Rösler IP GmbH, 88138 Hergensweiler (DE)
(72) Erfinder: Rösler, Peter, 88239 Wangen (DE); Rösler, Thiemo, 88239 Wangen-Neuravensburg (DE)
(74) Vertreter: Riebling, Peter

(56) Entgegenhaltungen:
- EP-A1- 2 279 708
- US-A- 5 538 428
- US-A1- 2009 065 387
- US-A1- 2014 042 050
- US-B1- 6 217 332
- US-B1- 6 280 192

## Beschreibung

Die Erfindung betrifft eine Verpackungshülse für medizinische Zwecke zur Aufbewahrung von sterilen Gegenständen nach dem Oberbegriff des Patentanspruches 1, und/oder Patentanspruches 2, und/oder Patentanspruches 12.

Bei der Aufbewahrung und Darreichung von medizinischen Gegenständen und Operationsinstrumenten im Operationssaal kommt es entscheidend darauf an, die Keimfreiheit des in einer Verpackungshülse angeordneten Gegenstandes oder Instrumentes zu gewährleisten.

Mit dem Gegenstand der DE 693 19 704 T2 ist eine sterilisierte Verpackung für chirurgische Geräte bekannt geworden, die aus einer äußeren Umhüllung, zum Beispiel einem Polyethylen-Beutel, besteht, der mit einem Abziehstreifen verschlossen ist. Im Inneren des Beutels sind mehrere Ablagen aus Schaumstoff mit geschlossenen Zellen angeordnet. In jede der Lagen aus Schaumstoff mit geschlossenen Zellen sind die verschiedenen Instrumente und Geräte zum Gebrauch beim chirurgischen Eingriff angeordnet. Der Behälter mit den Schaumstofflagen ist von einer ersten Umhüllung umgeben. Diese Umhüllung ist steril ausgebildet, und eine zweite sterile Umhüllung umgibt die erste sterile Umhüllung.

Ein solches Beutelsystem mit darin in Schaumstoff eingebetteten Instrumenten hat den Nachteil, dass seine Dichtheit nicht garantiert werden kann. Die Dichtheit wird nur durch Klebestreifen und zugeordneten Versiegelungen gewährleistet. Sie ist deshalb auch nicht zur Aufnahme von sterilen Flüssigkeiten geeignet, weil die Dichtheit der Verpackung nicht gewährleistet werden kann.

Die DE 699 38 259 C2 beschreibt eine sterile Verpackung für flexible Endoskope. Sie besteht aus einer Tasche mit einem vorderen Film aus einem klaren undurchlässigen Polymer und einem hinteren Film aus einem semipermeablen Material, wie zum Beispiel einem Polyethylen-Vlies. Eine Klebeversiegelung umspannt die Außenkanten des vorderen Films und bildet einen versiegelten Innenraum. Demnach besteht bei dieser Beutelverpackung wiederum der Nachteil, dass lediglich die Dichtheit durch Versiegelungsflächen im Bereich von Klebefilmen gewährleistet werden soll. Dies ist jedoch für bestimmte Anwendungszwecke, bei denen erhöhte Anforderungen an die Dichtheit gestellt werden, nicht ausreichend.

Die US 2014/0042050 A1 beschreibt eine Verpackungshülse für medizinische Zwecke zur Aufbewahrung von sterilen Gegenständen, die aus einem Dichtverschluss besteht, der auf eine einseitig offene Außenhülse der Verpackung aufschraubbar ist.

Im Dichtverschluss ist eine zweite Verpackungshülse klemmend aufgenommen, die als Innenhülse in die Außenhülse um 180 Grad versetzt einschiebbar und dort aufbewahrbar ist.

Im Innenraum der Innenhülse, die mit einem ähnlichen Dichtverschluss verschließbar ist, befindet sich der gegen Kontamination zu schützende Gegenstand.

Nachteil dieser bekannten Verpackung ist, dass mit dem Aufschrauben des Dichtverschlusses auf die Außenhülse nicht sichergestellt werden kann, dass die zugeordnete Dichtung auch ausreichend dicht ausgebildet ist.

Weiterer Nachteil ist, dass der vor Kontamination zu schützende Gegenstand nicht in der Innenhülse eingespannt ist. Er ist vielmehr frei verschiebbar. Die Entnahme eines solchen Gegenstandes ist deshalb schwierig, weil zur Entnahme der Dichtverschluss von der Innenhülse abgeschraubt werden muss, wodurch der dort lose aufbewahrte Gegenstand unbeabsichtigt herausfallen kann.

Nachteil der US 2014/0042050 A1 ist ferner, dass für eine betriebssichere Abdichtung des außenhülsenseitigen Dichtverschlusses auf der Außenhülse nicht gesorgt werden kann, weil die in der Bodenfläche des Dichtverschlusses aufgenommene Innenhülse nicht zu einer Verfestigung der Abdichtung zwischen dem Dichtverschluss und dem Innenraum der Außenhülse führt.

Weiterer Nachteil ist, dass auch der Dichtverschluss für den Verschluss der Innenhülse keine zuverlässige Abdichtung gewährleistet, denn auch bei diesem Dichtverschluss besteht das Problem, dass eine radiale Aufweitung der Dichtrippen dieses Dichtverschlusses durch einen in der Bodenfläche des Dichtverschlusses eingeklemmten Spreizgegenstand nicht gegeben ist.

Der Erfindung liegt deshalb ausgehend von der US 2014/0042050 A1 die Aufgabe zugrunde, eine Verpackungshülse für medizinische Zwecke zur Aufbewahrung von sterilen Gegenständen so weiterzubilden, dass die Handhabung der aufzubewahrenden sterilen Gegenstände wesentlich einfacher und betriebssicherer erfolgen kann, und dass stets für eine ausreichende Dichtung der Dichtverschlüsse auf der Außenhülse und der Innenhülse gesorgt ist.

Zur Lösung der gestellten Aufgabe ist die Erfindung durch die technische Lehre der unabhängigen s Anspruches 1 gekennzeichnet.

Die vorliegende Erfindung sieht in der Art der US 2014/0042050 A1 vor, dass zwei koaxial ineinander gesteckte Hülsen vorhanden sind, wobei eine Innenhülse um 180 Grad in ihrer Lage verdreht in den Innenraum der Außenhülse aufgenommen ist, und die Innenhülse mit ihrem bodenseitigen Teil in der bodenseitigen Aufnahme des Dichtverschlusses für den Verschluss der Außenhülse aufgenommen ist.

In der gleichen Weise, wie in der US 2014/0042050 A1 beschrieben, ist damit eine vereinfachte Handhabung möglich, weil in einem bevorzugten Ausführungsbeispiel die doppelte Verpackungshülse bestehend aus zwei koaxial ineinander abdichtend zusammen gesteckten und jeweils verschraubten Innen- und Außenhülsen zunächst außerhalb des sterilen Operationsraumes von einer Bedienungsperson geöffnet wird und die Innenhülse aus der Außenhülse heraus gezogen wird.

Die so frei gemachte Innenhülse mit dem dort eingebrachten medizinischen Gegenstand wird im verschlossenen Zustand durch eine Schleuse im Operationssaal gebracht und an die Operationsschwester und/oder Operateur übergeben.

Die Operationsschwester und/oder der Operateur öffnet unter sterilen Bedingungen die noch verschlossene Innenhülse, wobei der Dichtverschluss der Innenhülse aufschraubt und entfernt wird. Der in der Innenhülse aufbewahrte medizinische Gegenstand wird dann unter sterilen Bedingungen in den Operationsbereich eingebracht und in den menschlichen oder tierischen Körper eingesetzt.

In einer ersten Ausführung ist der zu haltende Gegenstand lose in der Innenhülse gelagert und kann durch Schütteln der geöffneten Innenhülse aus dieser entnommen werden, ohne den Gegenstand zu berühren.

Der Erfindung hat sich deshalb die Aufgabe gestellt, eine Verpackung für medizinische Zwecke zur Aufbewahrung von sterilen Gegenständen so weiterzubilden, dass wesentliche höhere Anforderungen an die Dichtheit erfüllt werden und ein besserer Schutz gegendurch Berührung erfolgte Kontamination gegeben ist.

Die Erfindung geht zunächst von einer Hartverpackung aus und vermeidet die eingangs genannten Beutelverpackungen. Deshalb beansprucht die Erfindung eine Verpackungshülse, die einseitig offen ist und mit einem Dichtverschluss verschließbar ist.

Ein solcher Dichtverschluss ist im Wesentlichen mit einem Dichtpfropfen oder Dichtstopfen vergleichbar, der in entweder auf das einseitig offene Ende der Verpackungshülse aufgeschraubt, aufgerastet, aufgeschoben oder in anderer Weise fest und abdichtend mit dem offenen Ende der Verpackungshülse verbunden werden kann, sodass die Verpackungshülse durch einen solchen Dichtverschluss verschlossen wird.

Bei der Verwendung derartiger Dichtverschlüsse ist es bekannt, am Außenumfang eines solchen stopfenartigen Dichtverschlusses ein oder mehrere Dichtrippen anzuordnen, die sich beim Einschrauben oder Einschieben des Dichtverschlusses in das einseitig offene Ende der Verpackungshülse an den Innenumfang der Verpackungshülse anlegen, und eine zusätzliche Abdichtung ergeben. Solche Dichtverschlüsse sind bei Röhrchen zur Aufbewahrung von Tabletten allgemein bekannt.

Damit können jedoch erhöhte Anforderungen an die Dichtheit eines solchen Dichtverschlusses mit am Außenumfang angeordneten Dichtrippen nicht erfüllt werden.

### Beschreibung einer ersten Ausführungsform

Die Erfindung sieht deshalb vor, dass der Dichtsitz der Dichtrippen des Dichtverschlusses am Innenumfang der Verpackungshülse dadurch verbessert wird, dass der Dichtverschluss eine einseitig offene bodenseitige Aufnahmeöffnung aufweist, die bevorzugt zylindrisch oder konisch sich erweiternd ausgebildet ist, und dass in diese Aufnahmeöffnung ein Spreizelement in axialer Richtung einschiebbar und/oder eindrehbar ist, welches im Klemmsitz von einer durch die Aufnahmeöffnung gebildeten Ringwand gehalten wird.

Sobald der Dichtverschluss auf die einseitig offene Ausnehmung in der Verpackungshülse aufgeschraubt und dadurch in die Verpackungshülse hinein geschoben wird, dringt auch das im Innenraum der Verpackungshülse angeordnete Spreizelement in die einseitig offene axiale Ausnehmung im Bodenbereich des Dichtverschlusses, und verformt die Ringwand radial nach außen, sodass sich die am Außenumfang der elastomer verformbaren Ringwand angeordneten Dichtrippen unter verstärktem Anpressdruck an die Innenseite der Außenhülse anpressen.

Somit wird mit der technischen Lehre des Anspruches 1 der Dichtsitz eines Dichtverschlusses in einer einseitig offenen Verpackungshülse in der Weise verbessert, dass im Innenraum der Verpackung ein Spreizelement angeordnet ist, welches beim Einsetzen, Einschrauben oder Einbringen des Dichtverschlusses in die Verpackungshülse in die einseitig offene bodenseitige Aufnahmeöffnung im Dichtverschluss eindringt, und die Wandungen des Dichtverschlusses radial nach außen verdrängt, und die am Außenumfang dieser Wandungen angeordneten Dichtrippen unter verstärktem Dichtungsdruck und Anpressdruck an die Innenseite der Außenhülse anpresst.

Ein solches Spreizelement kann eine später zu beschreibende Innenhülse oder ein steriler Gegenstand, insbesondere auch ein Operationswerkzeug oder ein sonstiges, steril zu haltendes Mittel sein, welches geeignet ist, mit einem Teil seines Körpers klemmend in die bodenseitig offene Öffnung des Dichtverschlusses einzudringen und diesen radial nach außen zu spreizen.

Dabei ist es in einer ersten Ausführung vorgesehen, dass das Spreizelement in axialer Richtung geradlinig in den Aufnahmeraum des Dichtstopfens eingeschoben und dort unter elastomerer Verformung des Dichtstopfens geklemmt wird.

In einer zweiten Ausführung ist es vorgesehen, dass das Spreizelement mit einer Dreh- und/oder Drehschubbewegung in die Aufnahmeöffnung am Dichtverschluss eindringt und sich dort mittels Reibschluss festlegt. Zu diesem Zweck können am Innenumfang der Aufnahmeöffnung im Dichtverschluss gewindeartige Vorsprünge, Noppen oder Gewindebahnen eingeformt sein, die mit zugeordneten gewindeartigen Vorsprüngen, Noppen, Gewindebahnen oder mit glatten Flächen am Außenumfang der Innenhülse zusammen wirken.

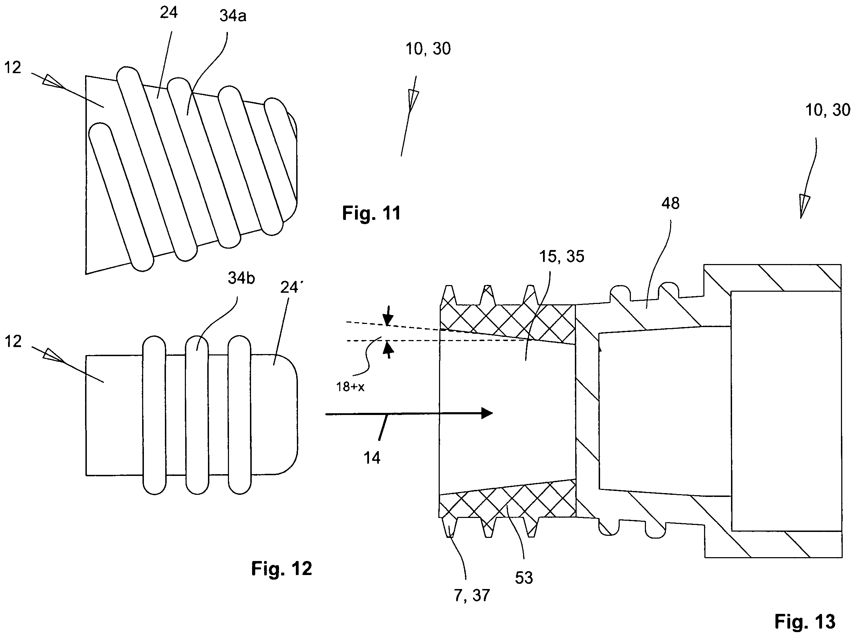

Wenn statt einer nur in axialer Richtung wirkenden Steck-Klemmverbindung nach der ersten Ausführung eine Dreh-Klemmverbindung nach der zweiten Ausführung gewählt wird, kann die Innenhülse mit einer kontrollierten Drehbewegung vom Dichtstopfen entfernt werden. Dies wird später anhand der Zeichnungsfiguren, Figuren 11 bis 13, erläutert werden.

In der einfachsten Ausführungsform der Erfindung besteht demnach die Verpackungshülse für medizinische Zwecke zur Aufbewahrung eines sterilen Gegenstandes aus einer Außenhülse, die einseitig offen ist und einen Dichtverschluss aufweist, wobei das Spreizmittel so ausgebildet sein soll, dass es mit einem Teil in die einseitig offene bodenseitige Öffnung des Dichtverschlusses klemmend und gegebenenfalls unter elastomerer Verformung eindringt, wenn der Dichtverschluss auf die Verpackungshülse aufgesetzt wird. Es kommt dabei zu einer radialen Aufweitung der Wandung des Dichtverschlusses, sodass die am Außenumfang des Dichtverschlusses angeordneten Dichtrippen unter erhöhtem Anpressdruck an die Innenwandung der Verpackungshülse angepresst werden.

In dieser vereinfachten Ausführungsform wird also lediglich eine Verpackungshülse mit einem Dichtverschluss und einem in die bodenseitig einseitig offene Aufnahmeöffnung eingreifenden Teil eines Spreizmittels beschrieben, wobei das Spreizmittel entweder durch den zu haltenden Gegenstand selbst gebildet ist oder durch die Bodenseite einer Innenhülse. Das Spreizmittel wird beim Einschrauben des Dichtverschlusses in die Außenhülse in die Aufnahmeöffnung des Dichtverschlusses klemmend aufgenommen und verformt elastisch die bodenseitige Aufnahmeöffnung im Dichtverschluss radial nach außen, sodass sich die am Außenumfang des Dichtverschlusses angeordneten Dichtrippen unter verstärktem Anpressdruck an die Innenseite der Außenhülse anlegen. Das Spreizelement wird somit entweder durch die Wandung der Innenhülse oder - bei Wegfall der Innenhülse - durch den zu haltenden Gegenstand selbst gebildet.

Der einfacheren Beschreibung wegen wird im Folgenden davon ausgegangen, dass die Innenhülse mit ihrem Bodenteil das Spreizelement für die Aufnahmeöffnung im Dichtverschluss der Außenhülse ist, obwohl die Erfindung nicht auf diesen Gegenstand beschränkt ist.

Vorteil dieser Maßnahme ist, dass eine einfache Entnahme der Innenhülse dadurch möglich ist, dass der äußere Dichtverschluss aus der Außenhülse heraus geschraubt oder gezogen wird, wobei die im Innenraum des äußeren Dichtverschlusses gehaltene Innenhülse aufgrund ihres Klemmsitzes im Dichtverschluss geklemmt bleibt, sodass beim Herausziehen oder Herausschrauben des Dichtverschlusses gleichzeitig auch die dort vom Dichtverschluss gehaltene Innenhülse aus der Außenhülse herausgezogen wird.

Der Dichtverschluss der Außenhülse dient deshalb als sterile Handhabe für die Handhabung der im Innenraum der Außenhülse angeordneten Innenhülse, und es besteht keine Notwendigkeit, die im Dichtverschluss klemmend aufgenommene Innenhülse mit der Hand zu berühren.

Somit betrifft die Erfindung eine Verpackungshülse aus zwei koaxial ineinander gesteckten Hülsen, nämlich einer von einem Dichtverschluss verschließbaren Außenhülse größeren Durchmessers und einer Innenhülse kleineren Durchmessers, die im Innenraum der Außenhülse aufgenommen ist und die an ihrem Ende durch einen zweiten Dichtverschluss verschließbar ist.

Somit sind in dieser Ausführungsform zwei unterschiedliche Hülsen ineinander gesteckt, denn im Innenraum der mit dem ersten Dichtverschluss verschließbaren Außenhülse wird die mit einem zweiten Dichtverschluss verschließbare Innenhülse im Klemmsitz in der bodenseitigen Aufnahmeöffnung des Dichtverschlusses der Außenhülse aufgenommen.

Die Innenhülse bildet demnach das Spreizelement für das Aufspreizen des Dichtverschlusses in der Außenhülse, und der dadurch gebildete Klemmsitz drückt die am Außenumfang des Dichtverschlusses angeordneten Dichtrippen unter verstärktem Anpressdruck an die Innenseite der Außenhülse.

Wenn in dieser Ausführungsform eine zweiteilige Verpackungshülse mit Innen- und Außenhülse vorgesehen ist, wird es bevorzugt, wenn die Innenhülse den zu haltenden, medizinischen Gegenstand beinhaltet.

Dadurch ergeben sich bei der Übergabe eines solchen medizinischen Gegenstandes im Operationssaal wesentliche Vorteile:
Im Prinzip liegt der Dichtverschluss, der die Außenhülse verschließt, dem Dichtverschluss gegenüber, der im Innenraum der Außenhülse angeordnet ist, sich an deren Bodenseite abstützt und der die Innenhülse unter verstärktem Anpressdruck abdichtet.

Zur Entnahme im Operationssaal ist es demnach vorgesehen, dass zunächst der erste Dichtverschluss, welcher die Außenhülse verschließt, aus der Außenhülse herausgezogen oder herausgeschraubt wird, wodurch die vom ersten Dichtverschluss im Klemmsitz gehaltene Innenhülse aus der Außenhülse herausgezogen werden kann.

Die Operationsschwester hält den ersten Dichtverschluss, der für den Verschluss der Außenhülse dient, in der Hand und reicht dem Operateur die im Klemmsitz im ersten Dichtverschluss aufgenommene Innenhülse, die noch durch den zweiten Dichtverschluss abdichtend verschlossen ist.

Der Operateur nimmt die Innenhülse mit dem noch darauf sitzenden zweiten Dichtverschluss entgegen und entfernt den zweiten Dichtverschluss aus der Innenhülse, wodurch das im Klemmsitz im zweiten Dichtverschluss aufgenommene Operationselement, Instrument oder Gegenstand im Klemmsitz am zweiten Dichtverschluss verbleibt, und - ohne dass der Operateur das Element, den Gegenstand oder das Instrument berühren muss - hält er den zweiten Dichtverschluss solange in der Hand, bis er - ohne Berührung des Elementes, Gegenstandes oder Instrumentes - das Instrument in den Operationsbereich einführen kann.

Statt den zu haltenden Gegenstand im Klemmsitz in der Innenhülse zu halten, kann es auch vorgesehen sein, den Gegenstand frei beweglich in der Innenhülse zu lagern.

Im ersten Fall ist eine berührungsfreie Manipulation des im Klemmsitz im Dichtverschluss der inneren Innenhülse klemmend aufgenommenen Gegenstandes, Elementes oder Instrumentes möglich.

Auch bei der Dichtungsanordnung zwischen dem zweiten Dichtverschluss und dem einseitig offenen Ende der Innenhülse ist in einer Weiterbildung der Erfindung gewährleistet, dass der zu haltende Gegenstand mit zum Beispiel seinem Kopfbereich im Klemmsitz in der bodenseitigen elastisch aufweitbaren Öffnung des zweiten Dichtverschlusses in der Innenhülse aufgenommen ist, sodass auch in diesem Fall durch die elastische Aufweitung der Aufnahmeöffnung die am Außenumfang des zweiten Dichtverschlusses angeordneten Dichtrippen gegen die Innenseite der Innenhülse unter verstärktem Anpressdruck angepresst werden.

Anstatt der Möglichkeit, dass der in der Innenhülse zu haltende Gegenstand selbst als Spreizelement für die Aufnahmeöffnung im Dichtverschluss der Innenhülse wirkt, kann es in einer anderen Ausführung vorgesehen sein, dass das Spreizelement in der Aufnahmeöffnung des inneren Dichtverschlusses als separates Druckstück ausgebildet ist.

Das Druckstück ist bevorzugt konisch ausgebildet und beim axialen Einschieben des Druckstücks in die gleichfalls konisch ausgebildete Aufnahmeöffnung wird diese radial aufweitet. Die axiale Längsbewegung des Druckstücks wird durch stirnseitige Anlage an dem in der Innenhülse lagengesichert gehaltenen Gegenstand erreicht.

### Beschreibung einer zweiten Ausführungsform

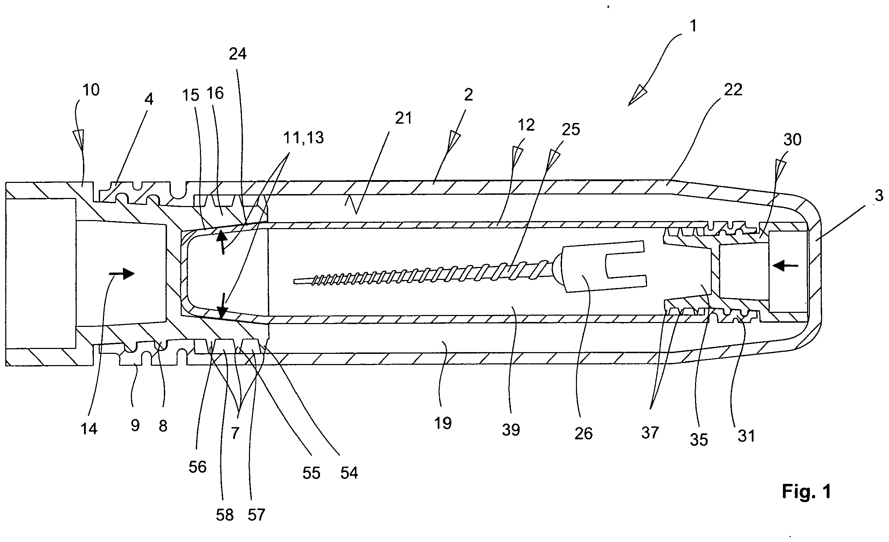

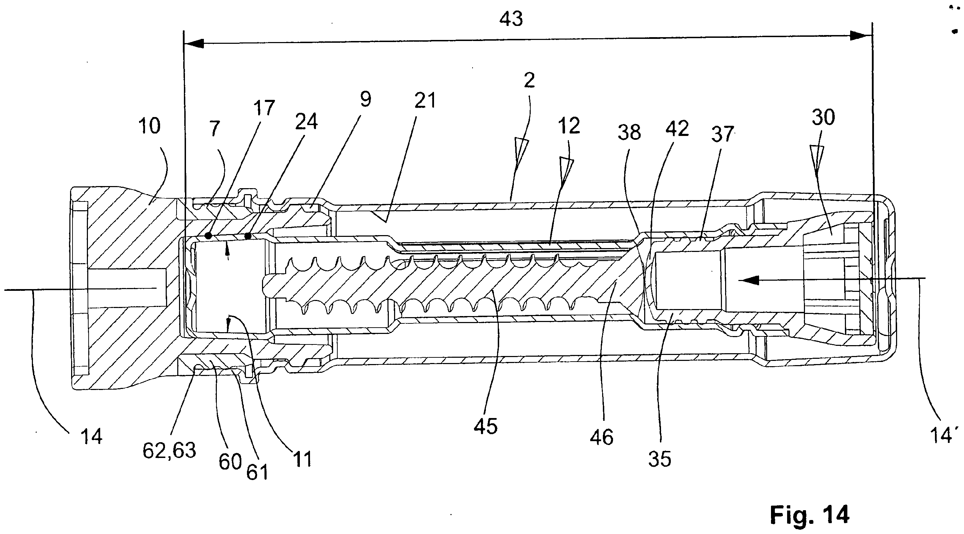

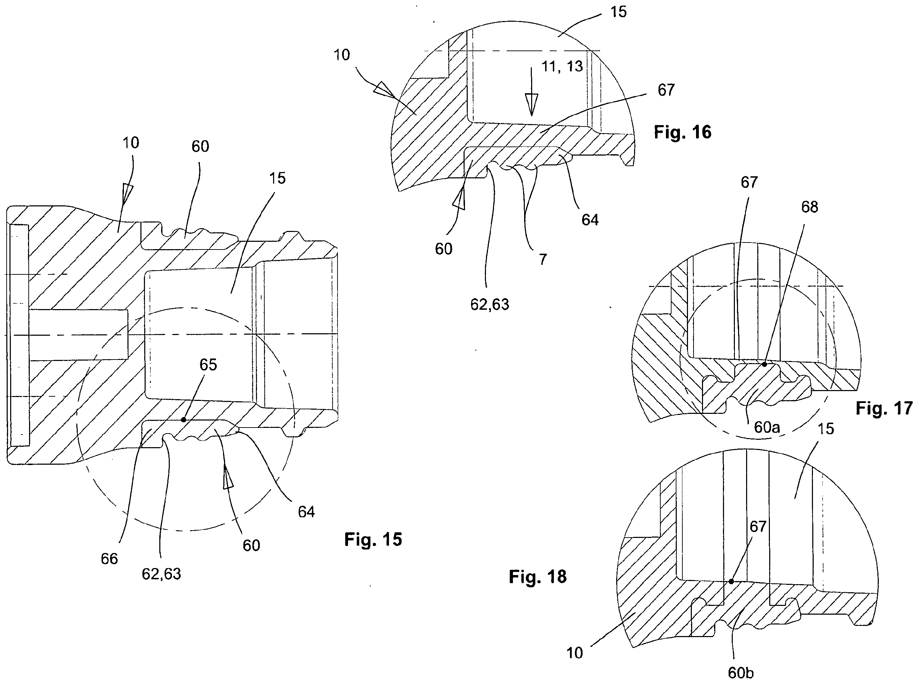

In einer zweiten Ausführungsform der vorliegenden Erfindung wird eine gegenüber der Ausführungsform nach Figur 1 bis 13 abgewandelte Ausführungsform beansprucht, wobei sich die zweite Ausführungsform - die insbesondere in Figur 15 und in den folgenden Figuren dargestellt ist, dadurch auszeichnet, dass eine andere Dichtungsanordnung für den Dichtverschluss vorhanden ist. Für die gleichen Teile des ersten Ausführungsbeispiels nach den Figuren 1-13 gelten die gleichen Bezugszeichen, Erläuterungen, Merkmale und Vorteile auch für die Teile des zweiten Ausführungsbeispiels nach Figur 14-19

Diese Anordnung zeichnet sich dadurch aus, dass gegenüber der Ausführungsform nach Figur 1 das Gewinde und die Dichtrippen gegeneinander vertauscht sind. Und ferner zeichnet sich das zweite Ausführungsbeispiel durch eine verbesserte Abdichtung dadurch aus, dass die Bodenhülse der inneren Hülse in Verbindung mit dem Dichtverschluss in einem anderen Dichtungszusammenhang steht, als vergleichsweise mit dem Ausführungsbeispiel nach Figur 1.

Nach der Ausführungsform gemäß der zweiten Ausgestaltung bildet das freie offene Ende der Außenhülse eine stirnseitig geöffnete Dichthülse, an deren Innenumfang nun ein neuartiger Dichtring ansetzt, der seinerseits am Außenumfang des neu ausgebildeten Dichtverschlusses sitzt.

Durch die Verwendung eines Dichtrings in Verbindung mit der Dichthülse am freien offenen Ende der Innenhülse ergibt sich eine besonders vorteilhafte Dichtung, wie sie bei dem ersten Ausführungsbeispiel nicht erreicht werden konnte.

Der Vorteil der zweiten Ausführungsform ist, dass die Dichtanordnung der Außenhülse in Verbindung mit dem Dichtstopfen in axialer Richtung ganz an die Stirnseite des Dichtstopfens gerückt ist, das heißt am axialen äußeren Ende des Dichtstopfens angeordnet ist, wodurch sich eine bessere Abdichtwirkung und ein verbesserter Schutz gegen Kontamination ergeben.

Der Bereich, in dem gedichtet wird und in dem der Dichtring angeordnet ist, ist somit soweit wie möglich in axialer Richtung auf den Stopfen mit dem Dichtverschluss verlegt. Damit ergibt sich der Vorteil, dass die Innensterilität der Außenhülse an allen Stellen gewährleistet ist.

Sollte es beim Entfernen des Dichtverschlusses zu einer unbeabsichtigten Berührung der Innenoberfläche der Außenhülse kommen, dann wird diese Kontamination nicht in den Innenraum der Außenhülse eingetragen, weil eine mögliche Kontamination nicht an die Innenfläche der Außenhülse gelangen kann. Somit werden eventuell kontaminierte Flächen axial soweit wie möglich von der Außenhülse entfernt angeordnet, um eine innenseitige Kontamination der Außenhülse zu vermeiden.

Die Erfindung beansprucht auch bestimmte Ausführungsformen für die Ausbildung der Abdichtung zwischen dem Dichtverschluss und dem Innenumfang der Außenhülse.

Hier wird in dem vorher erwähnten Ausführungsbeispiel ein Dichtring verwendet, der am Außenumfang des Dichtverschlusses aufgebracht ist und dort lagengesichert festgelegt ist. Dieser Dichtring weist unterschiedlich geformte Dichtrippen auf, und zwar eine stirnseitige Dichtrippe und zwei radial nach außen gerichtete Dichtrippen, sodass der Dichtring in zwei senkrecht zueinander gerichteten Richtungen - nämlich radial und axial - abdichtet.

Er ist ferner verdrehgesichert und gegen Verschiebung lagengesichert auf dem Dichtverschluss gehalten und ist deshalb unverlierbar.

In einer ersten Ausgestaltung ist die Lagensicherung des Dichtringes auf dem stopfenartigen Dichtverschluss so gewählt, dass er auf einem zylindrischen Außenumfang des Dichtverschlusses aufsitzt und eine relativ große Wanddicke radial einwärts in Richtung auf die Konusschräge des Dichtverschlusses ausbildet.

In einer anderen Ausgestaltung kann es jedoch vorgesehen sein, dass die Wanddicke im Bereich des Dichtstopfens an der Stelle, wo der Dichtring aufsitzt, stark vermindert ist, um zu gewährleisten, dass durch die verminderte Wanddicke ein Federelement gebildet wird und ein von innen nach außen gerichteter radialer Druck vom Dichtverschluss, der auf den Innenumfang des Dichtrings wirkt, diesen noch zusätzlich in radialer Richtung auswärts verformt und dadurch den Dichtungsdruck und die Dichtkraft am Innenumfang der Dichthülse im Bereich der Außenhülse noch verstärkt und verbessert.

Damit wird eine verbesserte Abdichtung erreicht, indem der Dichtring als Federelement ausgebildet ist, welches in der Lage ist, radiale Drücke von seinem Innenumfang auf den Außenumfang weiterzuleiten.

In einer weiteren Ausgestaltung kann es vorgesehen sein, dass der Dichtring werkstoffeinstückig mit dem stopfenartigen Dichtverschluss verbunden ist, und daher die vorher gegebene Wandstärke des Dichtrings vollständig entfällt. In diesem Fall bildet der erfindungsgemäße Dichtring unmittelbar eine Wandung des Dichtverschlusses aus und ist Teil dieser Wandung.
Er ist somit in der Lage, die auf die Innenseite des Dichtverschlusses einwirkende Verformungskraft unmittelbar radial auswärts auf die dort radial auswärts angeordnete Abdichtung weiterzuleiten.

Neben den vorher genannten drei Ausführungsbeispielen wird noch ein viertes Ausführungsbeispiel beschrieben, bei dem vorgesehen ist, dass der erfindungsgemäße Dichtring nicht an einer zylindrisch ausgebildeten Außenwand des stopfenartigen Dichtverschlusses anliegt, sondern dass diese Außenwand in axialer Richtung konisch zulaufend ausgebildet ist.

Genauer gesagt handelt es sich um zwei verschiedene Anlagewinkel, wobei in einem ersten Anlagebereich des Dichtrings eine zylindrische Anlagefläche und in einem zweiten Anlagebereich eine konische Anlagefläche gebildet ist.

Diese Art der Ausbildung der Anlageflächen hat den Vorteil, dass die Verspannungskraft zwischen der Innenhülse und dem Dichtverschluss an einer bestimmten definierten axialen Stelle am Dichtring eingeleitet wird, und dadurch eine überlegende Dichtwirkung erzielt wird, die konzentriert auf einen bestimmten Bereich der Abdichtung eingeleitet wird.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.

Alle in den Unterlagen, einschließlich der Zusammenfassung offenbarten Angaben und Merkmale, insbesondere die in den Zeichnungen dargestellte räumliche Ausbildung, werden als erfindungswesentlich beansprucht, soweit sie einzeln oder in Kombination gegenüber dem Stand der Technik neu sind.

Soweit einzelne Gegenstände als "erfindungswesentlich" oder "wichtig" bezeichnet sind, bedeutet dies nicht, dass diese Gegenstände notwendigerweise den Gegenstand eines unabhängigen Anspruches bilden müssen. Dies wird allein durch die jeweils geltende Fassung des unabhängigen Patentanspruches bestimmt.

Im Folgenden wird die Erfindung anhand von lediglich einen Ausführungsweg darstellenden Zeichnungen näher erläutert. Hierbei gehen aus den Zeichnungen und ihrer Beschreibung weitere erfindungswesentliche Merkmale und Vorteile der Erfindung hervor.

Es zeigen:
Figur 1: eine erste Ausführungsform der Erfindung im Längsschnitt
Figur 2: die Teile der Figur 1 in auseinandergezogener Darstellung
Figur 3: eine zweite Ausführung im Vergleich zur Figur 1
Figur 4: die Teile der Figur 3 in auseinandergezogener Darstellung
Figur 5: eine dritte Ausführung im Vergleich zur Figur 1
Figur 6: ein Schnitt durch einen Dichtverschluss in einer ersten Ausführung
Figur 7: ein Schnitt durch einen Dichtverschluss in einer zweiten Ausführung
Figur 8: Schnitt durch ein Druckstück in drei verschiedenen Ausführungen
Figur 9: eine Seitenansicht eines Dichtverschlusses
Figur 10: schematisiert ein Detailschnitt durch den Dichtverschluss mit 6 verschiedenen Darstellungen der Ausbildung der Dichtrippen
Figur 11: eine schematisierte Darstellung einer ersten Profilausbildung der Bodenseite der Innenhülse
Figur 12: eine schematisierte Darstellung einer zweiten Profilausbildung der Bodenseite der Innenhülse
Figur 13: schematisierte Darstellung des Eindringens der Bodenseite der Innenhülse in die Aufnahmeöffnung des Dichtverschlusses
Figur 14: eine gegenüber Figur 1 abgewandelte Ausführungsform
Figur 15: eine vergrößerte Darstellung eines stopfenartigen Dichtverschlusses mit der Anordnung des neuartigen Dichtrings gemäß Figur 14
Figur 16: eine detaillierte Darstellung gemäß Figur 15
Figur 17: eine gegenüber Figuren 15 und 16 abgewandelte Ausführungsform eines Dichtringes
Figur 18: eine zweite Abwandlung der Ausbildung des Dichtringes
Figur 19: eine Detaildarstellung der Ausbildung des Dichtringes gemäß Figur 17

Anhand der Figuren 1 bis 5 wird zunächst das Ausführungsbeispiel gemäß der technischen Lehre des unabhängigen Anspruches 1 beschrieben, wobei die Verpackungshülse 1 aus zwei koaxial ineinander gesteckten Verpackungshülsen 2, 12 besteht.

Die Verpackungshülse 1 besteht demnach aus einer zylindrischen Außenhülse 2 und einem die Öffnungsseite 4 der Außenhülse 2 verschließenden Dichtverschluss 10, sowie einer im Innenraum der Außenhülse 2 aufgenommenen Innenhülse 12, die in einer um 180 Grad gedrehten Lage im Innenraum 19 aufgenommen ist und ebenfalls einen Dichtverschluss 30 trägt.

Der außenhülsenseitige Dichtverschluss 10 weist ein Außengewinde 8 auf, welches mit einem zugeordneten Innengewinde 9 an der Öffnungsseite 4 der Außenhülse 2 zusammenwirkt. Auf diese Weise kann der Dichtverschluss 10 in die Öffnungsseite 4 der Außenhülse 2 eingeschraubt werden.

Am Außenumfang des Dichtverschlusses 10 sind im Bereich der axial vorderen, elastomer verformbaren Ringwand 16 und im axialen Abstand zum Außengewinde 8 eine Anzahl von parallel zueinander angeordneten Dichtrippen 7 angeordnet, die in Einschraubrichtung nach hinten geneigt sind und sich somit abdichtend an den Innenumfang 21 der Außenhülse 2 anlegen. Die durch die Ringwand 16 gebildete, einseitig offene Aufnahmeöffnung 15 ist als Konusschräge 17 mit einem Konuswinkel 18 ausgebildet.

Die Konusschräge 17 der Aufnahmeöffnung 15 kann glatt ausgebildet sein oder auch gewindeartig profiliert.

Die Öffnungsseite 4 der Außenhülse 2 ist durch einen Absatz 20 verringerten Durchmessers von dem übrigen Innenraum 19 der Außenhülse 2 abgesetzt, und die Bodenhülse 22 ist konisch verengt und bodenseitig geschlossen. Die Bodenhülse 22 ist somit durch die Bodenseite 3 abgeschlossen.

Die Stirnseite 5 der Öffnungsseite 4 ist offen und weist einen umlaufenden Rand auf, der im eingeschraubten Zustand des Dichtverschlusses 10 an einer zugeordneten, umlaufenden Anschlagkante 6 am Umfang des Dichtverschlusses 10 anschlägt.

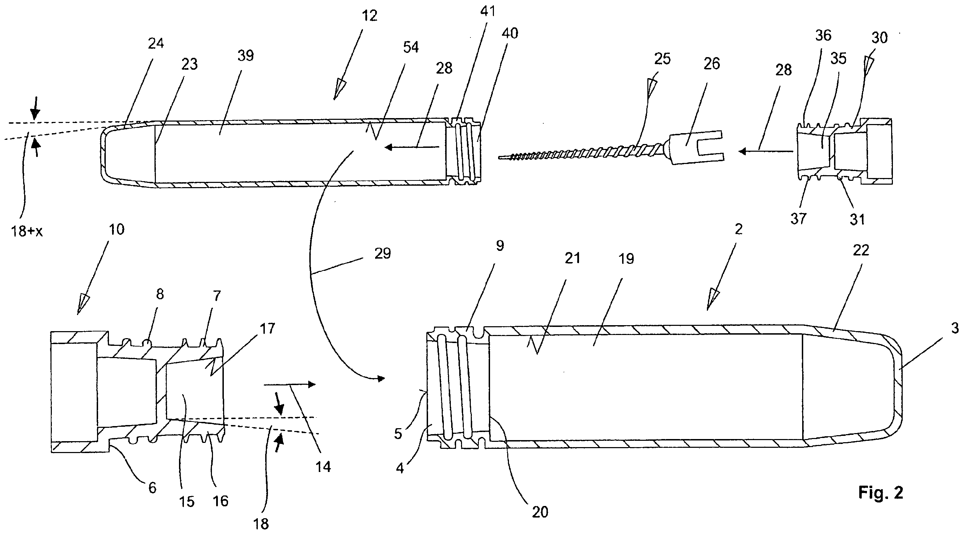

In Figur 2 sind alle Teile der Figur 1 in auseinandergezogener Darstellung gezeichnet, sodass deren gegenseitige Zuordnung erkennbar ist.

Der Dichtverschluss 30 weist - in der gleichen Weise wie der Dichtverschluss 10 - eine sich konisch nach außen erweiterte Aufnahmeöffnung 35 auf, die durch eine Ringwand 36 gebildet ist, an deren Außenumfang Dichtrippen 37 angeformt sind. Auch hier kann der Innenumfang der Aufnahmeöffnung 35 glatt oder gewindeartig profiliert sein.

Der Dichtverschluss 30 wird in Pfeilrichtung 28 auf die Öffnungsseite 40 der Innenhülse 12 aufgeschraubt. Es wird angenommen, dass der Gegenstand 25 mit seinem Kopf 26 schon im Innenraum 39 der Innenhülse 12 aufgenommen ist.

In manchen Anwendungsfällen kann es vorgesehen sein, dass der Durchmesser des Kopfes 26 des Gegenstandes 25 so gewählt ist, dass der Kopf 26 in der konisch sich erweiternden Aufnahmeöffnung 35 eingeklemmt ist und diese Aufnahmeöffnung 35 radial nach außen abspreizt, sodass sich die Dichtrippen 37 unter verstärktem Anpressdruck an den Innenumfang 54 der Innenhülse 12 anpressen.

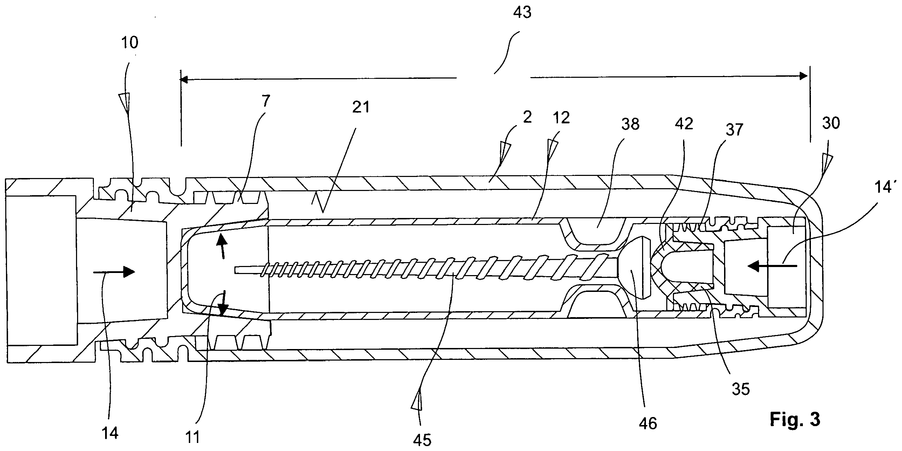

Die Figur 1 zeigt demnach, dass die Länge der Innenhülse 12 so gewählt ist, dass sich deren Dichtverschluss 30 an der Bodenseite 3 der Außenhülse 2 im Innenraum 19 abstützt, und die Länge 43 (siehe Figur 3) der Innenhülse 12 gewählt ist, dass die konisch zulaufende Bodenhülse 22 der Innenhülse 12 beim Einschrauben des Dichtverschlusses 10 in die Außenhülse 2 in Pfeilrichtung 14 in die Aufnahmeöffnung 15 im Dichtverschluss 10 eindringt und eine in radialer Pfeilrichtung 13 gerichtete Spreizbewegung ausführt, welche die umlaufende Ringwand 16 im Dichtverschluss 10 in Pfeilrichtung 13 radial nach außen verdrängt, und die Dichtrippen 7 unter verstärktem Anpressdruck an den Innenumfang 21 der Außenhülse 2 anpresst.

In Figur 1 sind die mehreren, aufeinanderfolgenden Dichtrippen allgemein mit dem Bezugszeichen 7 bezeichnet. Um zu zeigen, dass jede Dichtrippe umlaufend ist und in Verbindung mit der benachbarten Dichtrippe jeweils eine separate Dichtungskammer 57, 58 definiert, sind die einzelnen Dichtrippen mit separaten Bezugszeichen bezeichnet. Die axial äußerste Dichtrippe ist mit dem Bezugszeichen 56 bezeichnet, die in axialer Richtung darauf folgende Dichtrippe mit dem Bezugszeichen 57 und schließlich die darauf folgende Dichtrippe mit dem Bezugszeichen 59.

Die am Umfang in sich geschlossenen Dichtrippen 55, 56, 59 bilden die einzelnen Dichtungskammern 57, 58, die bei einem in radialer Pfeilrichtung 13 wirkenden Spreizdruck 11 komprimiert werden und dadurch wird die Abdichtung am Innenumfang 21 der Außenhülse 2 wesentlich verbessert.

Die Figur 2 zeigt, dass es bevorzugt wird, wenn die Aufnahmeöffnung 15 im Dichtverschluss 10 einen Konuswinkel 18 aufweist, und dass der Außenumfang der Bodenhülse 24 der Innenhülse 12 einen gleichen oder zumindest ähnlichen Konuswinkel 18 aufweist.

Die Konuswinkel 18 vom Dichtstopfen 10, 30 und der Bodenhülse 24 der Innenhülse 12 können jedoch auch voneinander abweichen.

Die Bodenhülse 24 der Innenhülse 12 ist demnach in der zentralen Aufnahmeöffnung 15 des Dichtverschlusses 10 geklemmt. Wenn der Dichtverschluss 10 aus der Außenhülse 2 herausgeschraubt ist, bleibt die Innenhülse 12 in der Aufnahmeöffnung 15 eingeklemmt.

Der hierdurch erzeugte Spreizdruck ist mit dem Bezugszeichen 11 in Figur 1 eingezeichnet.

Die Figur 1 zeigt auch, dass die Innenhülse 12 in einer um 180 Grad versetzten Lage zentrisch im Innenraum der Außenhülse 2 aufgenommen ist, und sich mit ihrem Dichtverschluss 30 an der Bodenseite 3 der Außenhülse 2 abstützt. Der Dichtverschluss 30 ist in gleicher Weise aufgebaut wie der Dichtverschluss 10, sodass für den Aufbau dieses Dichtverschlusses 30 alle vorher genannten Beschreibungen wie für den Dichtverschluss 10 gelten.

Vor dem sterilen Bereich des Operationssaales hat die Entnahme der Innenhülse 12 aus der Außenhülse 2 durch Öffnen des Dichtverschlusses 10 stattgefunden, wie in Figur 2 dargestellt. Dabei bleibt die Innenhülse 12 mit ihrer Bodenhülse 24 in der konischen Aufnahmeöffnung 15 des abgenommenen Dichtverschlusses 10 festgeklemmt. Die Handhabung erfolgt also nur durch Anfassen am Dichtverschluss 10 und die Innenhülse 12 wird nicht berührt.

Im Sterilbereich des Operationssaales öffnet eine geeignete Person gemäß Figuren 1 und 2 den Dichtverschluss 30 durch Abschrauben aus der Innenhülse 12, wobei der zu haltende Gegenstand 25 aus dem Innenraum 39 der Innenhülse herausgezogen wird. Er ist entweder lose in der Innenhülse 12 aufgenommen oder er ist mit seinem Kopf 26 klemmend in der Konusschräge 27 der Aufnahmeöffnung 35 gehalten.

Gemäß Figur 3 kann der Kopf 46 eines Gegenstandes 45 auch mithilfe eines im Dichtverschluss 30 angeordneten Druckstücks 42 gegen einen im Durchmesser verringerten Ringbund 38 der Innenhülse 12 festgeklemmt sein.

In allen drei Fällen kann nun der sterile Gegenstand 25, 45 aus der Innenhülse 12 entnommen werden und ohne weitere Berührung in das sterile Operationsfeld eingebracht werden.

Die Innenhülse 12 ist in gleicher Weise wie die Außenhülse 2 aufgebaut und weist einen an der Bodenseite angeordneten Absatz 23 auf, der in eine im Durchmesser konisch zulaufende Bodenhülse 24 übergeht.

Die Bodenhülse 24 ist das Spreizglied für das Aufspreizen der Aufnahmeöffnung 15 im Dichtverschluss 10. Im Bereich der Öffnungsseite 40 das Innengewinde 41 für das Einschrauben mit dem Außengewinde 31 am Außenumfang des Dichtverschlusses 30 angeordnet.

Ferner im Bereich der Ringwand 36 die am Außenumfang angeordneten Dichtrippen 37 angeordnet sind, die durch den Kopf oder ein anderes Halteelement des Gegenstandes 25 in radialer Richtung aufgespreizt werden.

Auch hier ist die Länge des Gegenstandes 25 so bemessen, dass er mit seinem vorderen Ende an der Bodenwand der Bodenhülse 24 anstößt, sodass beim Einschrauben des Dichtverschlusses 30 in die Innenhülse 12 der Kopf 26 in die konisch zulaufende Aufnahmeöffnung 35 eindringt und diese radial nach außen spreizt.

Selbstverständlich ist es in einer anderen Ausgestaltung auch möglich, die Länge des Gegenstandes 25 kürzer zu wählen und im Innenraum der Innenhülse axiale oder radiale Anschläge vorzusehen, an welchen der Gegenstand 25 lagengesichert anschlägt, sodass beim Einschrauben des Dichtverschlusses 30 in die Öffnungsseite 40 der Außenhülse 12 der Kopf 26 in die konisch zulaufende Aufnahmeöffnung 35 eindringt und diese aufspreizt.

Die Erfindung ist nicht auf die Halterung eines Gegenstandes in der Art einer Schraube - wie in den Zeichnungen dargestellt - begrenzt. Es kann jedes beliebige Instrument oder jeder beliebige andere Gegenstand 25 in der Innenhülse gehalten sein.

In einer anderen Ausgestaltung kann es auch vorgesehen sein, dass die Länge des Gegenstandes 25 kürzer ist als die Innenlänge der Innenhülse 12, sodass es keine axialen oder radialen Anschläge für den Gegenstand 25 braucht.

In diesem Fall kann es vorgesehen sein, dass der Gegenstand 25 mit seinem Spreizglied (zum Beispiel dem Kopf 26) bereits schon vornherein in die konische Aufnahmeöffnung 35 eingesteckt ist, die damit bereits aufgespreizt ist.

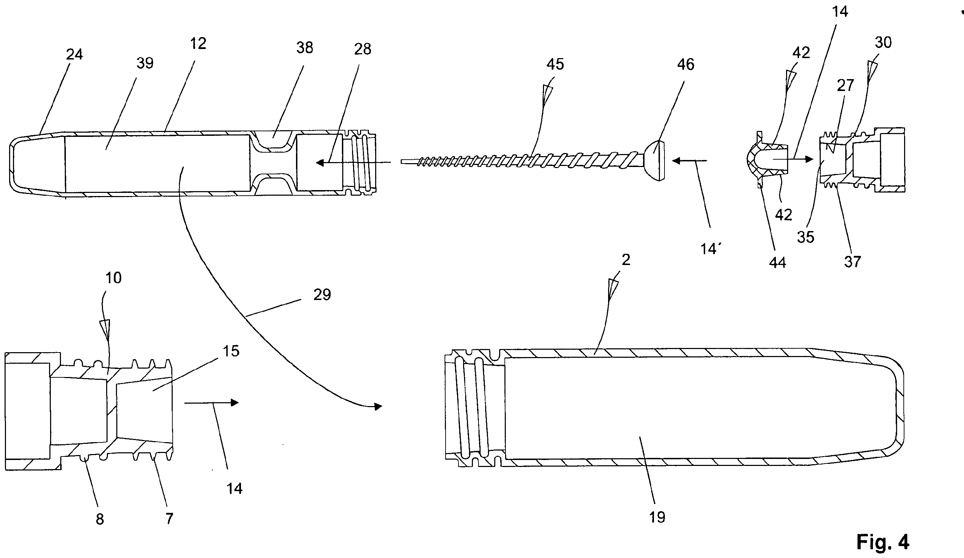

Die Figur 4 zeigt in auseinandergezogener Darstellung eine Anordnung einer Verpackungshülse, bestehend aus einer Außenhülse 2 und einer Innenhülse 12, bei der der zu verpackende Gegenstand 45 mit seinem Kopf 46 an einem Ringbund 38 verringerten Durchmessers im Innenraum 39 der Innenhülse 12 aufgenommen ist. Um den zu haltenden Gegenstand 45 gegen Bewegung im Innenraum 39 der Innenhülse 12 zu schützen, ist dieser mithilfe eines Druckstücks 42 festgespannt.

Bei der Einbringung des Gegenstandes 45 in die Innenhülse 12 muss die Innenhülse noch lose vorhanden sein oder nur vorläufig und mit geringer Einstecktiefe mit ihrem Druckstück 42 in die Aufnahmeöffnung 35 des Dichtverschlusses 30 eingesteckt sein.

Sobald jedoch der Dichtverschluss 30 mit seinem Außengewinde auf das zugeordnete Innengewinde der Innenhülse 12 aufgeschraubt wird, dringt das Druckstück 42 in Pfeilrichtung 14 weiter in den Innenraum der Aufnahmeöffnung 35 des Dichtverschlusses 30 ein, solange, bis sich der radial im Durchmesser erweiterte Anschlagrand 44 an der zugeordneten Stirnseite der Aufnahmeöffnung 35 anlegt. Somit führt das bevorzugt konisch ausgebildete Druckstück beim Eindringen in die Aufnahmeöffnung 35 des Dichtverschlusses 30 zu einem radialen Aufspreizen der Dichtrippen 37 und damit zu einer erhöhten Abdichtung des Dichtverschlusses 30 im Innenraum der Innenhülse 12.

Wie durch den Pfeil 29 in Figur 4 dargestellt, wird dann die so fertig montierte Innenhülse 12 mit dem nun unter axialem Druck festgeklemmten Gegenstand 45 in die Außenhülse 2 eingesetzt und die Gesamtlänge der Innenhülse 12 einschließlich des fertig montierten Dichtverschlusses 30 ist so gewählt, dass die konisch zulaufende Bodenhülse 24 der Innenhülse beim Einschrauben des Dichtverschlusses 10 auf die Außenhülse 2 in die konisch sich erweiternde Aufnahmeöffnung 15 eindringt und die Aufnahmeöffnung 15 radial aufweitet, sodass sich die Dichtrippen 7 unter verstärktem Anpressdruck an die Innenseite der Außenhülse 2 anlegen.

Die Erfindung ist nicht auf das in Figur 4 gezeigte Ausführungsbeispiel beschränkt, denn es kann in einer anderen - nicht zeichnerisch dargestellten Ausführung - vorgesehen sein, das Druckstück 42 auch für das Aufspreizen der Aufnahmeöffnung 15 im Dichtverschluss vorgesehen ist, genauso, wie es oben stehend anhand der Innenhülse 12 beschrieben wurde.

In diesem Fall wird dann nicht die konische Bodenhülse 24 der Innenhülse 12 in die Aufnahmeöffnung 15 eingreifen, sondern in die Aufnahmeöffnung 15 ist dann ein Druckstück 42 teilweise eingesteckt und beim Aufschrauben des Dichtverschlusses 10 dringt dieses Druckstück 42 vollständig in die Aufnahmeöffnung 15 des Dichtverschlusses 10 ein und weitet diesen ebenfalls auf.

Somit ist klargestellt, dass als Spreizglied für den äußeren Dichtverschluss 10 nicht nur die Bodenhülse 24 der Innenhülse 12 dienen kann, sondern dass der Dichtverschluss 10 auch durch ein nicht näher dargestelltes Druckstück 42 radial aufgespreizt werden kann, wie dies im Beispiel nach Figur 4 anhand des Dichtverschlusses 30 in Verbindung mit der Innenhülse 12 erläutert wurde.

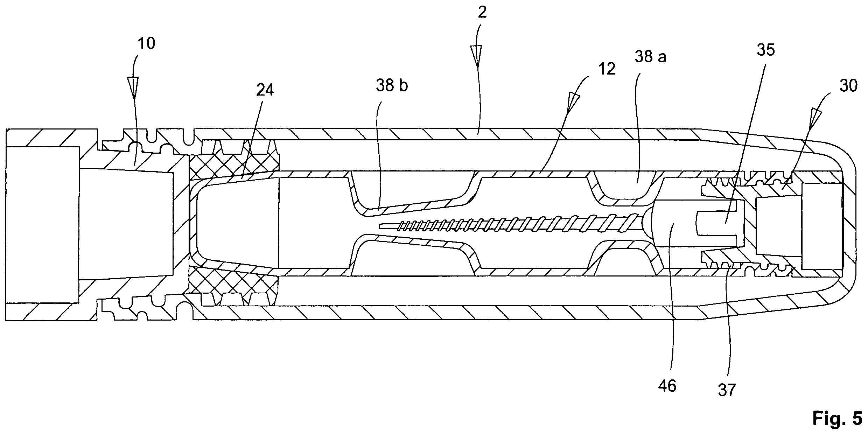

Die Figur 5 zeigt ein gegenüber Figur 4 abgewandeltes Ausführungsbeispiel, bei dem dargestellt ist, dass als Spreizglied für das Aufspreizen der Aufnahmeöffnung 35 im innenhülsenseitigen Dichtverschluss 30 auch unmittelbar der Kopf 46 eines zu haltenden Gegenstandes geeignet ist.

Ansonsten gelten für die gleichen Teile, wie in Figur 4 dargestellt, die gleichen Erläuterungen.

Die Figur 5 zeigt im Übrigen das Eindringen der Bodenhülse 24 nach Figur 4 in die Aufnahmeöffnung 15 des äußeren Dichtverschlusses 10. Die Figur 4 zeigt die auseinandergezogene Darstellung, während die Figur 5 die fertig montierte Darstellung zeigt, wobei die Dichtrippen 7 nun unter der Aufspreizwirkung der konischen Bodenhülse 24 mit vergrößertem Anpressdruck an dem Innenumfang der Außenhülse 2 angepresst sind.

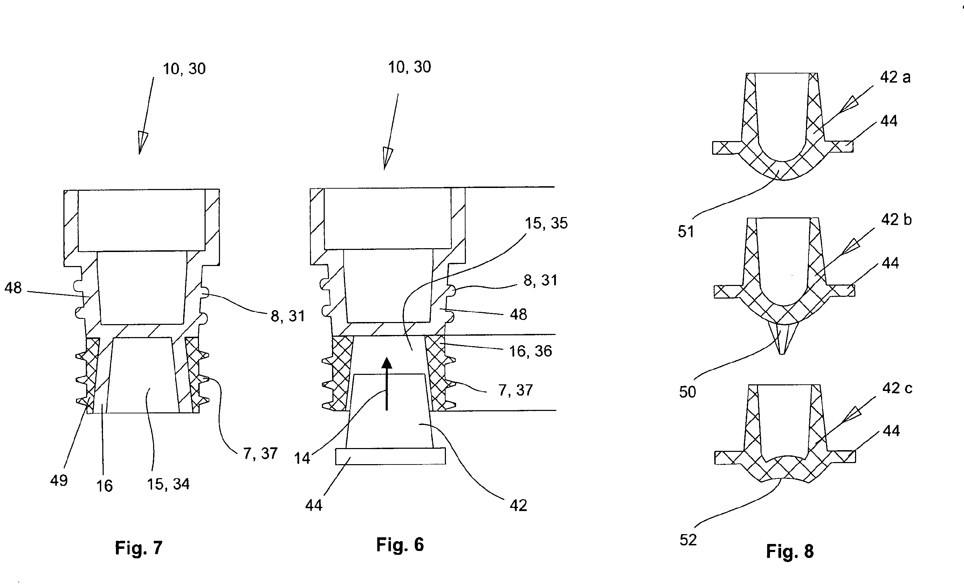

Die Figuren 6 und 7 zeigen verschiedene Ausführungen eines Dichtverschlusses, wobei jeder Dichtverschluss entweder für den äußeren Dichtverschluss 10 und/oder für den inneren Dichtverschluss 30 verwendet werden kann.

Die Figur 6 zeigt das Eindringen eines Druckstücks 42 mit einem konischen Teil in die konisch erweiterte Aufnahmeöffnung 15, 35 des Dichtverschlusses 10, der im gezeigten Ausführungsbeispiel zweiteilig ausgebildet ist.

Die Ringwand 16, 36, welche die Dichtrippen 7, 37 trägt, ist aus einem weicheren Kunststoffmaterial ausgebildet als vergleichsweise das darüber liegende Schraubteil 48, welches das Außengewinde 8 bzw. 31 aufweist.

Der in Figur 6 dargestellte Dichtverschluss 10, 30 besteht demnach aus zwei werkstoffeinstückig miteinander verbundenen Kunststoffmaterialien, wobei der Kunststoff der radial aufspreizbaren Ringwand 16, 36 aus einem weicheren Kunststoffmaterial als vergleichsweise der Kunststoff des darüber angeordneten Schraubteils 48 ist.

In Abwandlung der Ausführung nach Figur 6 zeigt die Figur 7, dass der gesamte Dichtverschluss 10, 30 aus einem härteren Kunststoff bestehen kann, der sich in der harten Ausführung auch bis in die Ringwand 16 hinein erstreckt.

Allerdings ist am Außenumfang der Ringwand 16 eine weichere Dichtungshülse 49 angeformt oder aufgestülpt, an deren Außenumfang die unter radialem Aufspreizdruck verformbaren Dichtrippen 7, 37 angeordnet sind.

Die Figur 8 zeigt drei verschiedene Ausführungsformen eines Druckstücks 42, dessen Funktion bereits schon anhand der Figur 4 erläutert wurde.

Die Formgebung des Druckstücks, insbesondere dessen Bodenseite, hängt vor allem von der Art des mithilfe des Druckstücks 42 einzuklemmenden Kopfes 46 eines Gegenstandes 45 ab.

In der oberen Darstellung nach Figur 8 ist das Druckstück 42a mit einer konvex nach außen gerichteten Bogenfläche 51 ausgerüstet, die - gemäß Figur 4 - einen gleichmäßigen Anpressdruck auf den Kopf 46 eines zu haltenden Gegenstandes 45 ausübt.

In der zweiten Darstellung in Figur 8 ist das Druckstück 42b mit einer Spitze 50 ausgerüstet, die in eine zugeordnete, nicht näher dargestellte Bohrung im Mittenbereich eines Kopfes 46 eines Gegenstandes 45 eindringt und so für eine zusätzliche Zentrierung eines zu haltenden Gegenstandes 45 im Bereich der Innenhülse 12 sorgt.

Eine gleiche Zentrierung ergibt sich durch die Konkavfläche 52 des Druckstückes 42c in Figur 8, weil an dieser Konkavfläche ebenfalls eine daran angepasste Kopffläche zentriert werden kann.

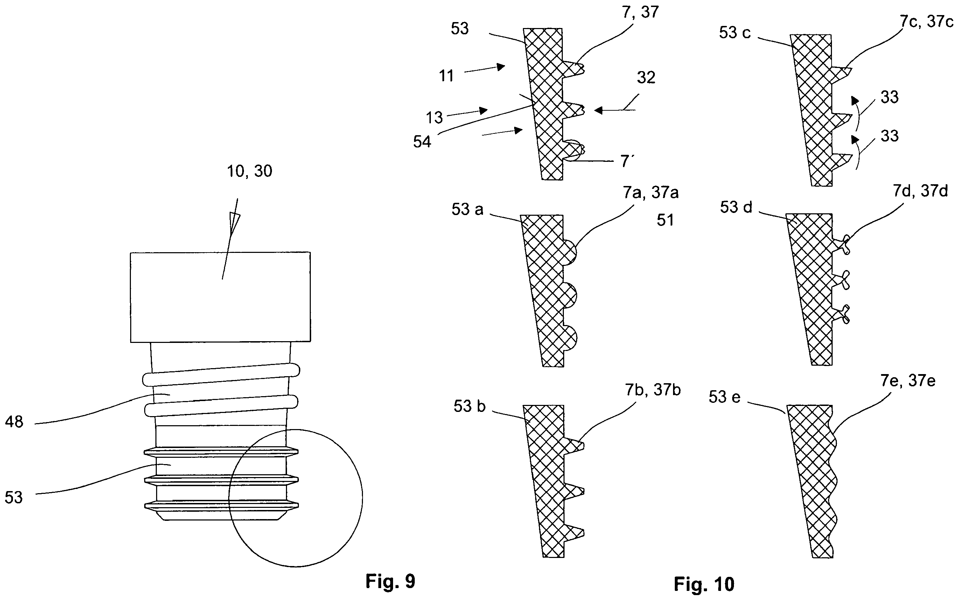

Die Figuren 9 und 10 zeigen unterschiedliche Ausbildungen von Dichtrippen 7, 37 eines Dichtverschlusses 10, 30. Es wird in Figur 9 angenommen, dass der Dichtverschluss aus dem oberen Schraubteil 48 und dem unteren Dichtungsteil 53 besteht, an dessen Außenumfang die unterschiedlichen Profilformen von Dichtrippen 7, 37 angeformt sind.

In der oberen Darstellung nach Figur 10 ist gezeigt, dass der in Pfeilrichtung 13 wirkende Spreizdruck 11 eines Spreizgliedes auf den Innenumfang 54 des Dichtungsteils 53 wirkt und damit zu einer radial nach außen gerichteten Verformung der Dichtrippen 7, 37 führt, weil diese unter Einwirkung eines Gegendrucks 32 durch Anlage am Innenumfang der Außenhülse 2 bzw. der Innenhülse 12 sich gleichmäßig radial einwärts verformen. Die etwa gleichmäßig symmetrisch und dreieckig profilierten Dichtrippen 7, 37 werden sich deshalb auch gleichmäßig unter Einwirkung des Gegendrucks 32 verformen, wie es anhand der Dichtrippe 7' in Figur 10 oben links dargestellt ist.

In der mittleren Darstellung der Figur 10 auf der linken Seite sind die Dichtrippen 7a, 37a bereits schon bogenförmig ausgebildet und werden sich deshalb unter Einwirkung eines Gegendrucks einwärts gerichtet verflachen und gerade mit großflächigen Dichtflächen an den Innenumfang der Außenhülse 2 oder der Innenhülse 12 anlegen.

Die untere Darstellung in Figur 10 auf der linken Seite zeigt, dass die Stirnseite der jeweiligen Dichtrippen 7b auch abgeflacht sein kann und nicht - gezackt wie in Figur 10 oben -, sodass sich eine andere Verformungsgeometrie unter Einfluss des Gegendrucks 32 ergibt.

In der rechten Darstellung nach Figur 10, rechts oben, werden die asymmetrisch ausgebildeten Dichtrippen 7c in Pfeilrichtung 33 verformt, da ein Gegendruck in Pfeilrichtung 32 auf die jeweilige asymmetrische Spitze der Dichtrippe 7c wirkt.

Im Unterschied zur rechten Darstellung oben in Figur 10 zeigt die mittlere Darstellung, dass die freien äußeren Enden der Dichtrippen 7d, 37d auch als radial nach außen vergrößerte Spitzen ausgebildet sein können, die sich dann unter Einwirkung eines Gegendrucks abflachen und damit geradlinige Anlageflächen am Innenumfang der Außenhülse 2 bzw. der Innenhülse 12 bilden.

Die untere Darstellung in Figur 10 auf der rechten Seite zeigt, dass es ausreicht, die Dichtrippen 7e, 37e auch nur bogenförmig auszubilden.

Wegen der Weichheit des verwendeten elastomeren Materials werden sich diese bogenförmigen Dichtrippen 7e, 37e ebenfalls gerade zusammendrücken und gerade, großflächige Anlageflächen unter Herstellung einer überlegenen Abdichtwirkung am Innenumfang der Außenhülse 2 bzw. der Innenhülse 12 entfalten.

Die Figur 11 zeigt als Ausführungsbeispiel eine Abwandlung der Formgebung des Außenumfanges der Bodenhülse 24 der Innenhülse 12.

Wie im allgemeinen Beschreibungsteil angegeben, kann es in einer abgewandelten Ausführungsform vorgesehen sein, dass der Außenumfang der konisch zulaufenden Bodenhülse 24 nicht glatt ausgebildet ist, sondern reibungserhöhende Profilelemente aufweist.

Diese Profilelemente 34a, 34b sollen eine gesteuerte Entnahme oder ein gesteuertes Herausziehen der Innenhülse 12 aus der Aufnahmeöffnung 15 im äußeren Dichtverschluss 10 ermöglichen. Beim Herausziehen der Bodenhülse aus der Aufnahmeöffnung 15 soll ein gesteuertes Herausziehen ohne große Krafteinwirkung möglich sein und die Ziehkraft soll möglichst kontrolliert ausgeübt werden.

So zeigt die Figur 11 am Außenumfang der konisch zulaufenden Bodenhülse 24 ein Schraubprofil 34a, sodass die Innenhülse 12 aus den Dichtstoffen 10 der Außenhülse 2 nicht mit einer axialen Zugkraft herausgezogen wird, sondern mit einer Schraubbewegung.

Damit ist eine gesteuerte Entnahme der Innenhülse 12 aus dem Dichtverschluss 10 der Außenhülse 2 möglich.

Dies ist im Übrigen auch in Figur 13 dargestellt, wo durch Eintragung von Bezugszeichen angegeben ist, dass die gleichen Merkmale auch für die Ausbildung des Dichtverschlusses 30 vorgesehen sein können, wobei dann - in Abwandlung der oben stehenden Beschreibung - entweder das Druckstück 42 der reibungserhöhenden Profile 34a, 34b aufweist oder, im Falle der Ausbildung nach Figur 5, wenn der Kopf 46 eines zu haltenden Gegenstandes unmittelbar als Spreizglied im Dichtstopfen 30 wirkt, dass dann der Kopf 46 mit den Profilformen 34a, 34b am Außenumfang ausgerüstet ist.

Der einfacheren Beschreibung wegen wird aber anhand der Figuren 11 bis 13 lediglich die Profilform am Außenumfang der Bodenhülse 24 der Innenhülse 12 diskutiert, obwohl die Erfindung nicht darauf beschränkt ist. Die Figur 12 zeigt in Abwandlung zur Figur 11, dass anstatt einer konisch zulaufenden Bodenhülse 24 auch eine zylindrische Bodenhülse verwendet werden kann, bei der zunächst die dargestellten Rippenprofile 34b entfallen. Sie ist dann glatt zylindrisch und kann ebenfalls unter Einwirkung einer Spreizkraft in die konisch sich verengende Aufnahmeöffnung 15 des Dichtverschlusses 10 eingeführt werden.

Eine verbesserte Spreizkraft ergibt sich jedoch dann, wenn am Außenumfang der zylindrischen Bodenhülse 24' einen größeren Durchmesser aufweisenden Rippen angeordnet sind.

Die Figur 13 zeigt, dass es nicht lösungsnotwendig ist, den Konuswinkel 18 der konisch zulaufenden Bodenhülse 24 mit dem Konuswinkel 18 der konisch sich verengenden Aufnahmeöffnung 15 in Übereinstimmung zu bringen.

Das Ausführungsbeispiel nach Figur 14 weist einen gleichen Dichtverschluss 30 auf, wie auch in der vorhergehenden Beschreibung anhand der Figuren 1 bis 13 beschrieben wurde.

Für die gleichen Teile wurden die gleichen Bezugszeichen verwendet. Es gelten auch alle Erläuterungen und Vorteile des ersten Ausführungsbeispiels.

Der wesentliche Unterschied zwischen dem Ausführungsbeispiel nach Figur 1 und Figur 14 ist die linksseitige Dichtungsanordnung, die nun erfindungsgemäß einen besonderen Dichtring 60 aufweist. Es wird jedoch darauf hingewiesen, dass der Dichtring 60 und seine besonderen Dichtfunktionen auch in Verbindung mit dem rechtsseitig dargestellten Dichtverschluss 30 verwendbar sind.

Der Dichtring 60 kann demnach linksseitig in Figur 14 bei dem Dichtverschluss 10 und/oder auch rechtsseitig in Figur 14 bei dem Dichtverschluss 30 angeordnet werden, wobei alle Informationen über die Art und Ausbildung des Dichtrings 60 auch für den rechtsseitigen Dichtverschluss 30 gelten.

Im Ausführungsbeispiel nach Figur 14 ist deshalb lediglich aus zeichnerischen Vereinfachungsgründen eine überlegende Dichtanordnung auf der linken Seite zwischen der Außenhülse 2 und dem stopfenartigen Dichtverschluss 10 dargestellt.

Diese Dichtanordnung zeichnet sich dadurch aus, dass sich der Dichtverschluss 10 mit seinem hülsenartigen Ende in den Innenraum der Außenhülse 2 erstreckt und dort ein Außengewinde 8 ausbildet, welches mit einem zugeordneten Innengewinde 9 auf der Innenseite der Außenhülse 2 zusammenwirkt.

Wichtig ist, dass der Gewindeeingriff zwischen den Gewinden 8, 9 axial einwärts auf den Innenraum der Außenhülse 2 verlegt ist, und axial auswärts nunmehr die Dichtanordnung auf der linken Seite der Gewindeanordnung 8, 9 ausgebildet ist.

Der Gewindeeingriff zwischen den einander zugeordneten Gewinden 8, 9 ist so gewählt, dass eine definierte Verrastposition in einer bestimmten Umdrehungslage erzielt wird, sodass damit gesorgt ist, dass der stopfenartige Dichtverschluss 10 stets in der gleichen Lagen- und Drehorientierung mit seinem Außengewinde 8 in das zugeordnete Innengewinde 9 der Außenhülse 2 eingeschraubt wird.

Das Ausführungsbeispiel nach Figur 14 zeichnet sich dadurch aus, dass es durch das ineinander Eindrehen der beiden zugeordneten Gewinde 8, 9 zu einer ersten axialen Dichtstelle 62 kommt, die am freien äußeren Ende einer Dichthülse 61 angeordnet ist, die werkstoffeinstückig mit der Wandung der Außenhülse 2 verbunden ist.

Die Dichtstelle 62 wird somit durch das stirnseitige Ende der außenwandseitigen Dichthülse 61 und einem Dichtring 60 gebildet, wobei der Dichtring 60 an der Dichtstelle 62 einen axialen Anschlag bildet und so die Dichtstelle 62 ausbildet.

Durch das Ineinanderdrehen der beiden Gewinde 8, 9 wird somit eine erste axiale Dichtung an der Dichtstelle 62 hergestellt.

Die Dichtstelle 62 wird demnach durch einen axialen Anschlag 63 im Bereich des Dichtringes 60 gebildet.

Im Ausführungsbeispiel nach Figur 15, welches eine vergrößerte Darstellung der Ansicht in Figur 14 ist, ist sichtbar, dass der Dichtring 60 ein etwa im Profil L-förmiges Teil ist, das heißt, es hat einen spitz zulaufenden, konischen Vorderbereich 64.

Es ist ferner eine innenseitige Anlagefläche 65 vorhanden, die im gezeigten Ausführungsbeispiel etwa zylindrisch ausgebildet ist.

Der Dichtring 60 bildet im Übrigen einen axialen Ringanschlag 66, der sich an einer zugeordneten axialen Anlagefläche am Kopf des stopfenartigen Dichtverschlusses 10 anlegt.

Gemäß Figur 16 ist der stirnseitige Anschlag 63 soweit wie möglich in axialer Richtung nach links in den Bereich des Dichtverschlusses 10 verlegt, während sich rechtsseitig in axialer Richtung an diesen axialen Anschlag 63 nunmehr die vorher beschriebenen radial nach außen gerichteten Dichtrippen 7 anschließen.

Die Dichtrippen sind jeweils etwa rund profilierte, rippenartige Körper, die werkstoffeinstückig mit dem Material des Dichtrings 60 ausgeformt sind und zwischen sich querschnittsverringernde Nuten aufweisen.

Es werden selbstverständlich auch alle anderen Ausführungsformen beansprucht, wie sie beispielweise in den Figuren 10ff. gemäß der vorstehenden Figurenbeschreibung erwähnt wurden.

Wie vorher beschrieben, wirkt nun im Innenraum der Konusschräge 17 der Innenhülse 12 ein radial nach außen gerichteter Spreizdruck 11 auf die Innenhülse 12 in Pfeilrichtung 13, und wird somit über die Hülsenwand 67 des stopfenartigen Dichtverschlusses 10 in radialer Richtung auf den neuartigen Dichtring 60 übertragen, der sich somit in zwei zueinander senkrechten Richtungen abdichtend an den Innenumfang der Außenhülse 2 anlegt.

Damit wird gewährleistet, dass der dichte und sterile Bereich möglichst weit in den Dichtring 60 in axialer Richtung nach außen verlegt ist, weil die Dichtstelle 62 an der weitest möglichen axialen Außenlage des Dichtrings 60 liegt, und sich erst danach - anschließend in axialer Richtung - die für die radiale Abdichtung vorgesehenen Dichtrippen 7 anschließen.

Dies ist im Unterschied zum Ausführungsbeispiel nach Figur 1, weil bei dieser Ausführungsform außerhalb des Dichtbereiches ein ungeschützter Innenbereich der Außenhülse vorhanden ist, der bei dem Ausführungsbeispiel nach Figur 14 ff. nicht mehr vorhanden ist.

Bei dem Ausführungsbeispiel nach Figur 17 wird eine andere Form eines Dichtrings 60 vorgeschlagen, der als Dichtring 60a bezeichnet ist.

Dieses Ausführungsbeispiel zeichnet sich dadurch aus, dass der Dichtring 60a keine zylindrische Anlagefläche 65 mehr aufweist, sondern diese Anlagefläche 68 ist nunmehr strukturiert ausgebildet.

Durch die strukturierte Anlagefläche ergibt sich eine Verringerung der Wanddicke 67 im Vergleich zu Figur 16, und somit wird deutlich, dass die Federwirkung der Hülsenwand 67 heraufgesetzt ist und sie in radialer Richtung den in Pfeilrichtung 13 ausgeübten Spreizdruck 11 besser auf den Dichtring 60a übertragen kann, der somit eine wesentlich stärkere Verformung in radialer Richtung auswärts erfährt.

Somit werden auch der Dichtungsdruck und die Dichtwirkung des Dichtrings 60a in Figur 17 verbessert.

Die Figur 18 zeigt in Abwandlung zu dem Ausführungsbeispiel nach Figur 17 eine andere Form eines Dichtrings, der deshalb als Dichtring 60b bezeichnet wird. Dieses Ausführungsbeispiel zeichnet sich dadurch aus, dass der Dichtring 60b in einem 2K-Verfahren als werkstoffeinstückiges Teil, jedoch mit anderem - weicheren - Elastizitätsmodul, mit dem Material des stopfenartigen Dichtverschlusses 10 zusammen als ein Teil gespritzt ist.

Er bildet also ein im Dichtverschluss 10 integrierter Körper, sodass bei diesem Ausführungsbeispiel die Hülsenwand 67 vollkommen entfällt, und die Hülsenwand 67 nun durch die Innenwand des Dichtringes 60b selbst gebildet wird.

Auf diese Weise wird dafür gesorgt, dass der Spreizdruck 11 in Pfeilrichtung 13 unmittelbar auf die Innenwand des Dichtrings 60b ausgeübt wird, der dadurch besonders empfindlich für radial auswärts gerichtete Verformungen ist.

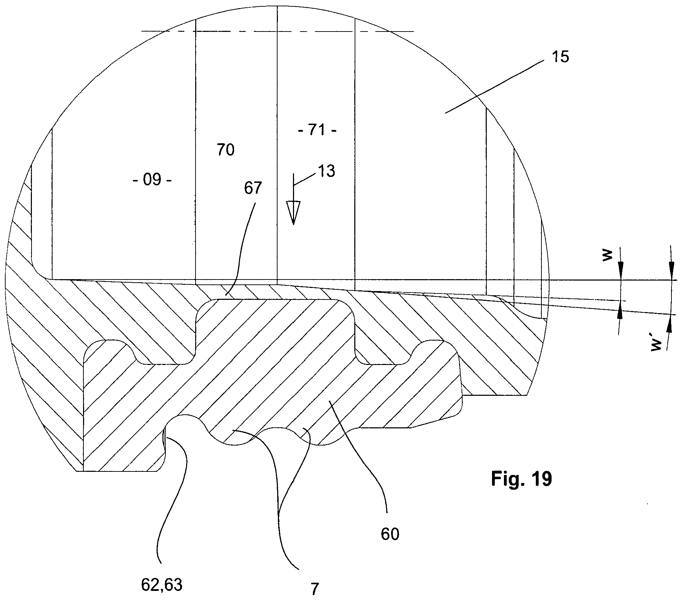

Die Figur 19 zeigt das Ausführungsbeispiel nach Figur 17 in vergrößerter Darstellung, wo erkennbar ist, dass die Hülsenwand 67 doppelt konisch ausgebildet ist.

Die Ausführungsbeispiele nach Figur 19 und Figur 17 stimmen in der Hinsicht überein, dass eine doppel-konische Aufnahmeöffnung 15 vorhanden ist. Sie besteht aus einem ersten Konusteil 69, einem zylindrischen Teil 70 und einem sich in axialer Richtung anschliessenden weiteren Konusteil 71.

Dementsprechend ist die Hülsenwand 67 in ihrer axialen Erstreckung in Bezug auf die Wanddicke abgestuft ausgebildet.
Sie ist gemäß der Darstellung in Figur 19 zunächst konisch zulaufend ausgebildet, und zwar um den Konuswinkel W, daran anschließend ist sie mit einem zylindrischen Teil 70 zylindrisch ausgebildet und bildet in axialer Verlängerung ab dem zylindrischen Teil 70 wiederum einen Konusteil 71, der den Konuswinkel W' aufweist, wobei W' größer ist als W.

Durch diese doppel-konische Ausbildung (W-Zylinder-W') ergibt sich der Vorteil, dass der Spreizdruck in Pfeilrichtung 13 besonders in den Bereichen 70 und 71 auf den Innenbereich des Dichtrings 60 übertragen wird, und somit eine Konzentration des Spreizdruckes in Pfeilrichtung 13 auf den Innenumfang des Dichtrings 60 stattfindet, der deshalb nur im Wesentlichen in diesem Bereich radial auswärts verformt wird, während die axiale Dichtstelle 62 von diesem radial nach außen gerichteten Spreizdruck unbeeinflusst bleibt.

Damit wird sichergestellt, dass die Dichtkraft hauptsächlich auf die radialen Dichtrippen 7 wirkt und weniger auf die axiale Dichtstelle 62.

Es kann vorgesehen sein, dass der Konuswinkel 18 um ein Maß X (das ist eine geradzahlige positive oder negative Zahl) von dem Konuswinkel der Bodenhülse 24 abweicht.

In allen Anwendungsfällen kommt es zu der gewünschten Aufspreizung des radial verformbaren Dichtungsteils 53, an dessen Außenumfang die elastomer verformbaren Dichtrippen 7, 37 angeformt sind.

### Zeichnungslegende

- 1: Verpackungshülse
- 2: Außenhülse
- 3: Bodenseite
- 4: Öffnungsseite
- 5: Stirnseite
- 6: Anschlagkante
- 7: Dichtrippen
- 8: Außengewinde
- 9: Innengewinde
- 10: Dichtverschluss (von 2)
- 11: Spreizdruck
- 12: Innenhülse
- 13: Pfeilrichtung
- 14: Pfeilrichtung
- 15: Aufnahmeöffnung (von 10)
- 16: Ringwand
- 17: Konusschräge
- 18: Konuswinkel
- 19: Innenraum (von 2)
- 20: Absatz
- 21: Innenumfang (von 2)
- 22: Bodenhülse (von 2)
- 23: Absatz
- 24: Bodenhülse (von 12)
- 24': Bodenhülse
- 25: Gegenstand
- 26: Kopf (von 25)
- 27: Konusschräge
- 28: Pfeilrichtung
- 29: Pfeilrichtung
- 30: Dichtverschluss (von 12)
- 31: Außengewinde (von 30)
- 32: Gegendruck
- 33: Pfeilrichtung
- 34a: Schraubprofil
- 34b: Rippenprofil
- 35: Aufnahmeöffnung (von 30)
- 36: Ringwand (von 30)
- 37: Dichtrippen (von 30)
- 38: Ringbund (von 12)
- 39: Innenraum (von 12)
- 40: Öffnungsseite
- 41: Innengewinde (von 12)
- 42: Druckstück (von 30)
- 43: Länge (von 12)
- 44: Anschlagrand
- 45: Gegenstand
- 46: Kopf (von 45)
- 47: Ringwand (von 42)
- 48: Schraubteil (von 10, 30)
- 49: Dichtungshülse (von 10, 30)
- 50: Spitze (von 42)
- 51: Bogenfläche (von 42)
- 52: Konkavfläche (von 42)
- 53: Dichtungsteil
- 54: Innenumfang
- 55: Dichtrippe
- 56: Dichtrippe
- 57: Dichtungskammer
- 58: Dichtungskammer
- 59: Dichtrippe
- 60: Dichtring a, b
- 61: Dichthülse
- 62: Dichtstelle (axial)
- 63: Anschlag (axial)
- 64: Vorderbereich (von 60)
- 65: Anlagefläche
- 66: Ringanschlag
- 67: Hülsenwand
- 68: Anlagefläche

## Patentansprüche

1. Verpackungshülse für medizinische Zwecke zur Aufbewahrung von sterilen Gegenständen (25, 45) bestehend mindestens aus einer einseitig offenen und mit einem Dichtverschluss (10) verschließbaren Außenhülse (2), in der eine, einen sterilen Gegenstand aufnehmende Innenhülse (12) klemmend aufgenommen ist, wobei die Innenhülse (12) im Klemmsitz in einer bodenseitigen Aufnahmeöffnung (15) des Dichtverschlusses (10) der Außenhülse (2) aufgenommen ist, **dadurch gekennzeichnet, dass** die Innenhülse (12) an deren geschlossenen Seite durch eine konisch verengte Bodenhülse (24) abgeschlossen ist, die in eine stirnseitig offene Aufnahmeöffnung (15) des Dichtverschlusses (10) der Außenhülse (2) eindringt und den dort vorhandenen Klemmsitz so verstärkt, dass die am Außenumfang des Dichtverschlusses (10) angeordneten Dichtrippen (7; 55, 56, 59) in radialer Richtung unter verstärktem Anpressdruck an die Innenseite der Außenhülse (2) gepresst sind, (Fig. 1).

2. Verpackungshülse für medizinische Zwecke zur Aufbewahrung von sterilen Gegenständen (25, 45) bestehend mindestens aus einer einseitig offenen und mit einem Dichtverschluss (10) verschließbaren Außenhülse (2), in der eine, einen sterilen Gegenstand aufnehmende, gleichfalls einen Dichtverschluss (30) aufweisende Innenhülse (12) klemmend aufgenommen ist, **dadurch gekennzeichnet, dass** der sterile Gegenstand (25, 45) im Klemmsitz in einer bodenseitigen Aufnahmeöffnung (35) des Dichtverschlusses (30) der Innenhülse (12) aufgenommen ist, und dass ein Kopf (46) des sterilen Gegenstandes (25, 45) unmittelbar selbst oder unter Zwischenschaltung eines Druckstücks (42) die am Außenumfang des Dichtverschlusses (30) angeordneten Dichtrippen (37) unter verstärktem Anpressdruck in radialer Richtung an die Innenseite der Innenhülse (12) anpresst, (Fig. 3, 4)

3. Verpackungshülse nach Anspruch 1 oder 2, **dadurch gekennzeichnet, dass** die einseitig offene, zylindrische Innenhülse (12) mit einem zweiten, stopfenförmigen Dichtverschluss (30) verschließbar ist und an der gegenüberliegenden Seite durch eine Bodenhülse (24) verschlossen ist, die klemmend in der Aufnahmeöffnung (15) des Dichtstopfens (10) der Außenhülse (2) gehalten ist, und dadurch den Dichtstopfen (10) radial aufweitet, (Fig. 1).

4. Verpackungshülse nach einem der Ansprüche 1 bis 3, **dadurch gekennzeichnet, dass** mindestens der erste Dichtverschluss (10) die einseitig offene, bodenseitige Aufnahmeöffnung (15) aufweist, in welche entweder der zu haltenden Gegenstand (25, 45) oder die Bodenhülse (24) der Innenhülse (12) klemmend aufgenommen ist, wobei ferner der Durchmesser der Bodenhülse (24) der Innenhülse (12) oder der Durchmesser des zu haltenden Gegenstandes (25, 45) so ausgebildet sind, dass beim Einstecken der Bodenhülse (24) in die Aufnahmeöffnung (15) im ersten Dichtverschluss (10) oder beim Einstecken des zu haltenden Gegenstandes (25, 45) die Aufnahmeöffnung (15) radial nach außen gespreizt wird und sich die am Außenumfang am ersten Dichtverschluss (10) angeordneten Dichtrippen (7; 55, 56, 59) unter verstärktem Anpressdruck an den Innenumfang (21) der Außenhülse (2) anlegen.

5. Verpackungshülse nach einem der Ansprüche 1 bis 4, **dadurch gekennzeichnet, dass** auch die Innenhülse (12) mit einem Dichtverschluss (30) verschließbar ist, der eine einseitig offene Aufnahmeöffnung (35) für die klemmende Halterung des medizinischen Gegenstandes (25, 45) aufweist.

6. Verpackungshülse nach Anspruch 5, **dadurch gekennzeichnet, dass** bei der klemmenden Halterung des Gegenstandes (25, 45) in der einseitig offenen Aufnahmeöffnung (35) des zweiten Dichtverschlusses (30) der mit Dichtrippen (37) besetzte Außenumfang des Dichtverschlusses (30) an den Innenumfang (54) der Innenhülse (12) mit verstärktem Anpressdruck anpressbar ist.

7. Verpackungshülse nach einem der Ansprüche 1 bis 6, **dadurch gekennzeichnet, dass** der erste Dichtverschluss (10) und/oder der zweite Dichtverschluss (30) jeweils in die zugeordnete Hülse (2, 12) einschraubbar und/oder einsteckbar und/oder einrastbar sind.

8. Verpackungshülse nach einem der Ansprüche 1 bis 7, **dadurch gekennzeichnet, dass** auf der hülsenartigen, einseitig offenen bodenseitigen Aufnahmeöffnung (15) des Dichtverschlusses (10) am oberen axialen Ende mindestens ein Dichtring (60, 60a, 60b) angeordnet ist, an den sich in axialer Richtung das Gewinde (8) anschließt, (Fig. 14-19)..

9. Verpackungshülse nach einem der Ansprüche 1 bis 8, **dadurch gekennzeichnet, dass** das Material des Dichtverschlusses (10, 30) im Bereich der Dichtrippen (7; 55, 56, 59; 37) aus einem weichen Kunststoff und das Material im Bereich des Gewindeteils (8, 31; 34) aus einem harten Kunststoffmaterial besteht.

10. Verpackungshülse nach einem der Ansprüche 1 bis 9, **dadurch gekennzeichnet, dass** die einseitig offene Aufnahmeöffnung (15, 35) als in axialer Richtung sich erweiternde Konusöffnung (17, 18) ausgebildet ist.

11. Verpackungshülse nach einem der Ansprüche 1 bis 10, **dadurch gekennzeichnet, dass** mindestens in der Aufnahmeöffnung (35) des zweiten Dichtverschlusses (30) ein die Aufnahmeöffnung (35) unter axialer Vorspannung radial aufweitendes Druckstück (42) angeordnet ist, welches sich am zu haltenden Gegenstand (25, 45) unter Vorspannung anlegt.

12. Verpackungshülse nach einem der Ansprüche 1 bis 11, **dadurch gekennzeichnet, dass** der Dichtverschluss (10, 30) aus einem Schraubteil (48) und einem damit werkstoffeinstückig verbundenen Dichtungsteil (53) besteht und dass am Außenumfang des Dichtungsteils (53) die sich unter radialem Druck verformenden Dichtrippen (7, 37) angeordnet sind.

13. Verpackungshülse nach Anspruch 12, **dadurch gekennzeichnet, dass** der Dichtungsteil (53) zweiteilig ausgebildet ist und aus einer härteren, inneren Ringwand (16) und einer die Ringwand (16) radial außen umgebenden Dichtungshülse (49) aus einem weicheren Material besteht, an deren Außenumfang die elastomer verformbaren Dichtrippen (7; 55, 56, 59; 37) angeordnet sind.

14. Verfahren zum Betrieb einer Verpackungshülse nach einem der Ansprüche 1 oder 2, bestehend mindestens aus einer einseitig offenen und mit einem Dichtverschluss (10) verschließbaren Außenhülse (2), in der eine Innenhülse (12) klemmend aufgenommen ist, **dadurch gekennzeichnet, dass** beim Einschrauben des Dichtverschlusses (10) in das Gewinde (9) der Außenhülse (2) zunächst eine radiale und axiale Abdichtung (62, 63) zwischen dem Dichtverschluss (10) und der Außenhülse (2) hergestellt wird und erst danach der Gewindeeingriff (8,9) vollendet wird. (Fig. 14-19).

## Claims

1. Packaging sleeve for medical purposes for storing sterile objects (25, 45) consisting of at least one outer sleeve (2) which is open at one end and can be closed with a sealing closure (10), and in which an inner sleeve (12) accommodating a sterile object is accommodated with a clamping effect, wherein the inner sleeve (12) is accommodated in a clamping seating in a bottom-side receiving opening (15) of the sealing closure (10) of the outer sleeve (2), **characterised in that** the inner sleeve (12) is sealed on its closed side by a conically narrowed base sleeve (24) which penetrates into a receiving opening (15), which is open on the end-face side, of the sealing closure (10) of the outer sleeve (2) and increases the clamping seating present there so that the sealing ribs (7; 55, 56, 59) arranged on the outside circumference of the sealing closure (10) are pressed against the inside of the outer sleeve (2) in radial direction under increased contact pressure, (Figure 1).

2. Packaging sleeve for medical purposes for storing sterile objects (25, 45) consisting of at least one outer sleeve (2) which is open at one end and can be closed with a sealing closure (10), and in which an inner sleeve (12) accommodating a sterile object and likewise having a sealing closure (30) is accommodated with a clamping effect, **characterised in that** the sterile object (25, 45) is accommodated in a clamping seating in a bottom-side receiving opening (35) of the sealing closure (30) of the inner sleeve (12), and **in that** a head (46) of the sterile object (25, 45) directly itself or with interposition of a pressure piece (42) presses the sealing ribs (37) arranged on the outside circumference of the sealing closure (30) against the inside of the inner sleeve (12) under increased contact pressure in radial direction, (Figures 3, 4).

3. Packaging sleeve according to claim 1 or 2, **characterised in that** the cylindrical inner sleeve (12), which is open at one end, can be closed with a second stopper-type sealing closure (30) and is closed on the opposite side by a bottom sleeve (24), which is held with a clamping effect in the receiving opening (15) of the sealing stopper (10) of the outer sleeve (2) and thereby widens the sealing stopper (10) radially, (Figure 1).

4. Packaging sleeve according to one of claims 1 to 3, **characterised in that** at least the first sealing closure (10) has the bottom-side receiving opening (15) which is open at one end and into which either the object (25, 45) to be held or the bottom sleeve (24) of the inner sleeve (12) is accommodated with a clamping effect, wherein also the diameter of the bottom sleeve (24) of the inner sleeve (12) or the diameter of object (25, 45) to be held are designed so that on insertion of the bottom sleeve (24) into the receiving opening (15) in the first sealing closure (10) or on insertion of the object (25, 45) to be held, the receiving opening (15) is spread radially outward and the sealing ribs (7; 55, 56, 59) arranged on the outside circumference on the first sealing closure (10) are in contact with the inside circumference (21) of the outer sleeve (2) under increased contact pressure.

5. Packaging sleeve according to one of claims 1 to 4, **characterised in that** the inner sleeve (12) can also be closed with a sealing closure (30), which has a receiving opening (35), which is open at one end for the clamping hold of the medical object (25, 45).

6. Packaging sleeve according to claim 5, **characterised in that** in clamping hold of the object (25, 45) in the receiving opening (35), which is open at one end, of the second sealing closure (30), the outside circumference of the sealing closure (30), which is provided with sealing ribs (37), can be pressed against the inside circumference (54) of the inner sleeve (12) with increased contact pressure.

7. Packaging sleeve according to one of claims 1 to 6, **characterised in that** the first sealing closure (10) and/or the second sealing closure (30) can be screwed and/or inserted and/or engaged respectively into the assigned sleeve (2, 12).

8. Packaging sleeve according to one of claims 1 to 7, **characterised in that** at least one sealing ring (60, 60a, 60b), to which the thread (8) is connected in axial direction, is arranged on the sleeve-type, open at one end bottom-side receiving opening (15) of the sealing closure (10) on the upper axial end, (Figures 14-19).

9. Packaging sleeve according to one of claims 1 to 8, **characterised in that** the material of the sealing closure (10, 30) in the area of the sealing ribs (7; 55, 56, 59; 37) consists of a soft plastic, and the material in the area of the thread part (8, 31; 34) consists of a hard plastic material.

10. Packaging sleeve according to one of claims 1 to 9, **characterised in that** the receiving opening (15, 35), which is open at one end, is designed as a conical opening (17, 18) which becomes wider in the axial direction.

11. Packaging sleeve according to one of claims 1 to 10, **characterised in that** a pressure piece (42), which radially widens the receiving opening (35) under axial prestress and is in contact with the object (25, 45) to be held under prestress, is arranged at least in the receiving opening (35) of the second sealing closure (30).

12. Packaging sleeve according to one of claims 1 to 11, **characterised in that** the sealing closure (10, 30) consists of a screw part (48) and a sealing part (53) which is connected thereto in one material piece, and **in that** the sealing ribs (7, 37), which undergo deformation under radial pressure, are arranged on the outside circumference of the sealing part (53).

13. Packaging sleeve according to claim 12, **characterised in that** the sealing part (53) is designed in two parts and consists of a harder, inner annular wall (16) and a sealing sleeve (49) made of a softer material, surrounding the annular wall (16) on the outside radially, on the outside circumference of which the elastomerically deformable sealing ribs (7; 55, 56, 59; 37) are arranged.

14. Method for operating a packaging sleeve according to one of claims 1 or 2, consisting of at least one outer sleeve (2) which is open at one end and can be closed with a sealing closure (10) and in which an inner sleeve (12) is accommodated with a clamping effect, **characterised in that** when the sealing closure (10) is screwed into the thread (9) on the outer sleeve (2), first a radial and axial seal (62, 63) is established between the sealing closure (10) and the outer sleeve (2) and only then is the thread engagement (8, 9) completed. (Figures 14-19).

## Revendications

1. Étui d'emballage à des fins médicales, pour conserver des objets stériles (25, 45), composé au moins d'un étui extérieur (2) qui est ouvert d'un côté, qui est apte à être fermé avec une fermeture d'étanchéité (10), et dans lequel un étui intérieur (12) recevant un objet stérile est reçu par serrage, l'étui intérieur (12) étant reçu dans une ouverture de réception (15), située côté fond, de la fermeture d'étanchéité (10) de l'étui extérieur (2), en étant calé par serrage, **caractérisé en ce que** l'étui intérieur (12) est obturé, sur son côté fermé, par un étui de fond (24) de forme rétrécie conique, qui entre dans une ouverture de réception (15), ouverte côté frontal, de la fermeture d'étanchéité (10) de l'étui extérieur (2) et qui renforce le calage par serrage existant à cet endroit, de telle sorte que les nervures d'étanchéité (7 ; 55, 56, 59) disposées sur la circonférence extérieure de la fermeture d'étanchéité (10) sont pressées dans le sens radial avec une pression d'application renforcée contre le côté intérieur de l'étui extérieur (2) (fig. 1).

2. Étui d'emballage à des fins médicales, pour conserver des objets stériles (25, 45), composé au moins d'un étui extérieur (2) qui est ouvert d'un côté, qui est apte à être fermé avec une fermeture d'étanchéité (10), et dans lequel un étui intérieur (12) recevant un objet stérile et comportant également une fermeture d'étanchéité (30) est reçu par serrage, **caractérisé en ce que** l'objet stérile (25, 45) est reçu en étant calé par serrage dans une ouverture de réception (35), située côté fond, de la fermeture d'étanchéité (30) de l'étui intérieur (12), et **en ce qu'**une tête (46) de l'objet stérile (25, 45) presse, directement elle-même ou par l'intermédiaire d'une pièce de pression (42), les nervures d'étanchéité (37) disposées sur la circonférence extérieure de la fermeture d'étanchéité (30), avec une pression d'application accrue dans le sens radial contre le côté intérieur de l'étui intérieur (12) (fig. 3, 4).

3. Étui d'emballage selon la revendication 1 ou 2, **caractérisé en ce que** l'étui intérieur (12), cylindrique et ouvert d'un côté, est apte à être fermé avec une seconde fermeture d'étanchéité en forme de bouchon (30) et est fermé sur son côté opposé par un étui de fond (24) qui est retenu par serrage dans l'ouverture de réception (15) du bouchon d'étanchéité (10) de l'étui extérieur (2), et qui élargit ainsi radialement ledit bouchon d'étanchéité (10) (fig. 1).

4. Étui d'emballage selon l'une des revendications 1 à 3, **caractérisé en ce qu'**au moins la première fermeture d'étanchéité (10) comporte l'ouverture de réception (15) ouverte d'un côté et prévue côté fond, dans laquelle soit l'objet à retenir (25, 45), soit l'étui de fond (24) de l'étui intérieur (12) est reçu par serrage, le diamètre de l'étui de fond (24) de l'étui intérieur (12) ou le diamètre de l'objet à retenir (25, 45) étant par ailleurs étudié pour que lors de l'introduction de l'étui de fond (24) dans l'ouverture de réception (15) prévue dans la première fermeture d'étanchéité (10), ou lors de l'introduction de l'objet à retenir (25, 45), l'ouverture de réception (15) soit élargie par écartement radialement vers l'extérieur et que les nervures d'étanchéité (7 ; 55, 56, 59) disposées sur la circonférence extérieure de la première fermeture d'étanchéité (10) s'appliquent avec une pression accrue contre la circonférence intérieure (21) de l'étui extérieur (2) .

5. Étui d'emballage selon l'une des revendications 1 à 4, **caractérisé en ce que** l'étui intérieur (12) est lui aussi apte à être fermé avec une première fermeture d'étanchéité (30) qui présente une ouverture de réception (35), ouverte d'un côté, pour la fixation par serrage de l'objet médical (25, 45).

6. Étui d'emballage selon la revendication 5, **caractérisé en ce que** lors de la fixation par serrage de l'objet (25, 45) dans l'ouverture de réception (35), ouverte d'un côté, de la seconde fermeture d'étanchéité (30), la circonférence extérieure, pourvue de nervures d'étanchéité (37), de ladite fermeture d'étanchéité (30) est apte à être pressée avec une pression d'application accrue contre la circonférence intérieure (54) de l'étui intérieur (12).

7. Étui d'emballage selon l'une des revendications 1 à 6, **caractérisé en ce que** la première fermeture d'étanchéité (10) et/ou la seconde fermeture d'étanchéité (30) sont aptes à être vissées et/ou introduites et/ou enclenchées dans l'étui associé (2, 12), respectivement.

8. Étui d'emballage selon l'une des revendications 1 à 7, **caractérisé en ce que** sur l'ouverture de réception (15), en forme d'étui, ouverte d'un côté et située côté fond, de la fermeture d'étanchéité (10), est disposée sur l'extrémité axiale supérieure au moins une bague d'étanchéité (60, 60a, 60b) à laquelle fait suite, dans le sens axial, le filetage (8) (fig. 14-19).

9. Étui d'emballage selon l'une des revendications 1 à 8, **caractérisé en ce que** le matériau de la fermeture d'étanchéité (10, 30) se compose, dans la zone des nervures d'étanchéité (7 ; 55, 56, 59 ; 37), d'une matière plastique souple et, dans la zone de la partie filetée (8, 31 ; 34), d'un matériau en matière plastique dur.

10. Étui d'emballage selon l'une des revendications 1 à 9, **caractérisé en ce que** l'ouverture de réception (15, 35) ouverte d'un côté est conçue comme une ouverture conique (17, 18) qui va en s'élargissant dans le sens axial.

11. Étui d'emballage selon l'une des revendications 1 à 10, **caractérisé en ce qu'**il est prévu au moins dans l'ouverture de réception (35) de la seconde fermeture d'étanchéité (30) une pièce de pression (42) qui élargit radialement ladite ouverture de réception (35), avec une précontrainte axiale, et qui s'applique contre l'objet (25, 45) à retenir, avec une précontrainte.

12. Étui d'emballage selon l'une des revendications 1 à 11, **caractérisé en ce que** la fermeture d'étanchéité (10, 30) se compose d'une partie à vis (48) et d'une partie d'étanchéité (53) reliée d'une seule pièce à celle-ci, et **en ce que** sur la circonférence extérieure de la partie d'étanchéité (53) sont disposées les nervures d'étanchéité (7, 37) qui se déforment sous une pression radiale.

13. Étui d'emballage selon la revendication 12, **caractérisé en ce que** la partie d'étanchéité (53) est en deux parties et se compose d'une paroi annulaire intérieure (16), plus dure, et d'un étui d'étanchéité (49) en matériau, plus souple, qui entoure ladite paroi annulaire (16) radialement vers l'extérieur et sur la circonférence extérieure duquel sont disposées les nervures d'étanchéité (7 ; 55, 56, 59 ; 37) aptes à subir une déformation élastomère.

14. Procédé pour le fonctionnement d'un étui d'emballage selon l'une des revendications 1 ou 2, composé au moins d'un étui extérieur (2) qui est ouvert d'un côté, qui est apte à être fermé avec une fermeture d'étanchéité (10), et dans lequel un étui intérieur (12) est reçu par serrage, **caractérisé en ce que** lors du vissage de la fermeture d'étanchéité (10) dans le filetage (9) de l'étui extérieur (2), une étanchéité radiale et axiale (62, 63) est tout d'abord réalisée entre la fermeture d'étanchéité (10) et l'étui extérieur (2), et après seulement, l'accouplement par filetage (8, 9) est achevé (fig. 14-19).
